# EUROPEAN PATENT APPLICATION

(11) **EP 4 389 802 A1**
(43) Date of publication of application: **26.06.2024**
(21) Application number: 22215477.5
(22) Date of filing: 21.12.2022
(51) Int. Cl.: C08J 3/12, A61K 8/02, A61K 8/73, A61Q 19/00

(54) **NOVEL AND VERSATILE BIODEGRADABLE MICROPOWDER**

(71) Applicant: Bioweg UG, 49610 Quakenbrück (DE)
(72) Inventor: Mittal, Anuj, 49610 Quakenbrück (DE); Mahalwar, Prateek, 49610 Quakenbrück (DE); Karaturi, Srinivas, 49610 Quakenbrück (DE)
(74) Representative: Hertin und Partner Rechts- und Patentanwälte PartG mbB

(57) **Abstract**

The invention preferably relates to a micropowder having an oil absorption capacity of at least 2.5 g/g, wherein the micropowder is biodegradable. The invention preferably relates further to a biodegradable micropowder comprising bacterial cellulose particles, wherein an average particle diameter of the bacterial cellulose particles is between 1 - 1000 µm and an average crystallinity of the bacterial cellulose particles is between 30 - 80%. The invention also preferably relates to a method for producing a micropowder. The method comprises the steps of producing bacterial cellulose from a bacterial culture such that an average crystallinity of the bacterial cellulose produced is between 30 - 80%, grinding bacterial cellulose to form bacterial cellulose particles with an average particle diameter of 1 - 1000 µm and drying the ground bacterial cellulose particles. The invention also relates to a cosmetic or personal care product comprising the micropowder.

## Description

The invention preferably relates to a micropowder having an oil absorption capacity of at least 2.5 g/g, wherein the micropowder is biodegradable. The invention preferably relates further to a biodegradable micropowder comprising bacterial cellulose particles, wherein an average particle diameter of the bacterial cellulose particles is between 1 - 1000 µm and an average crystallinity of the bacterial cellulose particles is between 30 - 80%. The invention also preferably relates to a method for producing a micropowder. The method comprises the steps of producing bacterial cellulose from a bacterial culture such that an average crystallinity of the bacterial cellulose produced is between 30 - 80%, grinding bacterial cellulose to form bacterial cellulose particles with an average particle diameter of 1 - 1000 µm and drying the ground bacterial cellulose particles. The invention also relates to a cosmetic or personal care product comprising the micropowder.

### BACKGROUND AND PRIOR ART

The invention belongs to the technical field of micropowders and microbeads, in particular biodegradable micropowders. More particularly the invention belongs to the technical field of biodegradable micropowders suitable for use as rheology modifiers, viscosity enhancers, texture enhancers, texturizing agents, sensory enhancers or opacifiers as solids or with a variety of organic and inorganic media.

The use of micropowders and microbeads to adjust aesthetic, sensory, rheological properties, hydrophobicity, hydrophilicity, oil absorption capacities, UV-absorption capacities or other functional properties of a solid or liquid product is known. Such powders and microbeads are currently used in a variety of products, for example in personal care, home care, dental care, pharmaceutical products, coatings and paint formulations. These are typically manufactured from non-biodegradable synthetic polymers as well as from minerals and metal oxides.

In the case of liquid products, sunscreens and foundation creams may be taken as an example. Titanium dioxide (TiO₂) particles which contribute to the opacity and sun-filtering properties are often provided in powder form and suspended in the formulation to adjust its color and sunabsorbance. Such formulations are often stored for months before use, during which time the suspended particles may settle, resulting in inconsistencies in the color and opacity of the product. To avoid this, the suspended particles are ground to a very small diameter and combined with rheology modifiers which slow down any settling behavior. Furthermore, the adherence of the formulation to the skin and the avoidance of unwanted wetness, greasiness or stickiness depends on the absorptive capacities of the formulation. As the oil and water absorption capacity of the titanium oxide particles is low, additional microbeads made for example from talcum powder or plastic may be added.

As an example of solid products, cosmetic pressed powders may be considered. Cosmetic pressed powders are often based on a pulverized mineral material such as talc, silica or a metal such as magnesium. In some cases, microbeads are also important components of such products. Titanium dioxide and zinc oxide particles are common ingredients added to adjust the opacity of the product or to obtain a sufficient sun protection. Metallic, mineral or plastic powders are often also used to provide a desired pigment to such cosmetic pressed powders. In addition to the aesthetic qualities, there is now also a high consumer demand for such products to provide a soft, non-greasy finish, to fill pores and/or to comfortably remain on the skin for long periods of time despite sebum production. These textural properties are usually provided by fine plastic powders comprising materials such polymethyl methacrylate or trimethylol hexyllactone.

A major drawback of these known micropowders is their lack of biodegradability which is now a pressing environmental and public health issue. There is ample evidence that microplastics have been consumed by fish which has found its way to the market. Such microplastics can travel quickly through the human body and are suspected of being able to cross the bloodbrain barrier, causing neurological problems and impacting on the human immune system. There is therefore an urgent need to develop biodegradable and biocompatible alternatives to the existing powders currently in widespread use.

Moreover, concerns have been raised in recent years over the safety of many micropowder components typically used in various consumer products. Talcum for instance - a common ingredient in pressed powders, deodorants and baby care products - has been suspected of being cancerous due to its presence being found in tumors. As talcum naturally occurs on the earth's surface close to other known carcinogenic minerals such as asbestos, there is a fear of cross-contamination occurring during its mining. This could lead to asbestos entering the body as part of such talcum powders.

Very fine particles of aluminum (10 - 50 µm) or aluminum salts are also used in deodorants to improve the feeling of dryness and to provide an anti-perspirant effect by blocking pores. The small size of the particles is such that their absorption capacity is high, they can be easily dispersed in a spray and they are not perceptible to the user. Their small size however allows them to overcome tissue barriers and to enter the human body. In recent times, a link has been made between breast cancer and the use of aluminum-based deodorants. It is thought that the fine aluminum particles enter the lymph nodes and travel to the breast tissue, causing an increase in tumors observed close to the lymph nodes. As the particles are not broken down by the body, they can accumulate, posing a risk to the surrounding tissue. There is therefore a need to replace these powders with safer, more biocompatible alternatives.

In addition, there has been a recent consumer and regulatory push to replace synthetic ingredients - not only in cosmetics but in all consumer products - with raw materials of natural origin or to develop formulations composed partially or fully of natural ingredients. There is therefore a need for viable alternatives which are not only biodegradable and safer than the current options but can also compete with consumer demands regarding the aesthetic quality, feel and function of products. In particular, there is a demand for a versatile biodegradable micropowder which can fulfil the function of several powder types - from opacity to oil absorption and soft texture.

The use of natural polymers to replace the non-natural polymers of non-biodegradable powders has been considered. For example, US2011274629A1 teaches the use of modified xanthan gum and galactomannan as a dry powder blend for making personal care products. While the powder blend was found to provide adequate thickening of lotions and the like, the powder blend could not compete with the versatile functions of conventional microplastics. The application of the dry powder blend in a solid (rather than emulsified or liquid) final product has not been investigated. Consumers currently have high expectations of cosmetic and personal care products which are worn on the skin. These should ideally have a soft, natural-looking texture without being dry, cakey or flakey. Skin products can flake when they do not adequately adhere to the skin or when they cannot absorb a layer of sebum building up between the product and the skin. This is thought to be due to the limited absorption capacities of known biodegradable alternatives to metallic and plastic microbeads. There is therefore a need for a micropowder which is not only biodegradable but also provides a long-lasting soft texture and a matte look when worn.

Plant cellulose has also been considered as a potential substitute for synthetic non-biodegradable powder materials. Cellulose is usually produced from wood pulp and is bound with components such as wax, lignin, pectin and hemicelluloses. These additional components and the chemical structure of plant cellulose limit its hydrophilicity and its solubility in aqueous media. There are therefore challenges in the production of smooth emulsions from plant cellulose. Moreover, its natural off-white color makes it less desirable for use in paints or white products where metal oxides are still preferred. Additionally, products which include powders based on plant cellulose have so far not been found to meet up to consumer demands regarding soft texture and non-greasy feel.

Surface-modified bacterial celluloses have also been used for their oil absorbing capacities outside the field of consumer products. For example, CN103962105A discloses a PTES (phenyltriethoxysilane) surface modified bacterial cellulose aerogel oil-absorbing material for use in cleaning oil spills. The gel is made by breaking a bacterial cellulose film, mixing the broken film with deionized water and freeze-drying for 24 - 72 hours to produce an aerogel. The aerogel is then functionally modified in ethanol in order to increase its hydrophobicity. It is this functional modification which is credited with allowing the achievement of the claimed oil absorption capacities of up to 50 g/g. While this indicates that there are naturally-based materials with high oil absorption properties, it does not provide a substitute for the known micropowders and microbeads. A gel is a homogeneous structure relying on networking effects forming between long fibers. Micropowders and microbeads on the other hand are preferably formed as discrete particles. Agglomeration of the particles is usually not desired. Especially in the case of scrubs and exfoliants, hardened, distinct particles are needed to fulfil the scrubbing function.

The high hydrophobicity and the modified chemical structure of the aerogel may be suitable for oil spills but not for use on skin, where chemical stability, non-stickiness and non-greasiness are desired. In addition to these limitations, the surface-modification of cellulose with PTES makes the resulting gel petroleum-based, such that it is no longer biodegradable. Microbeads used on the skin should ideally be fully biodegradable and do not accumulate in the body. There is thus a need for a micropowder whose material and properties are suitable for use with a variety of everyday consumer products.

There is a need for an alternative micropowder which is versatile in its properties. Preferably the micropowder is biodegradable and is harvested from renewable sources. The micropowder is preferably a good opacifier, of neutral color, can provide advantageous rheological properties and is biologically safe. It is especially preferable that the micropowder does not pose a health risk to humans if it enters the food chain. To viably replace synthetic powders used in cosmetics and personal care products, there is a need for the biodegradable powder to provide a soft, non-sticky and non-greasy feel on the skin.

### SUMMARY OF THE INVENTION

An objective of the present invention was to overcome the drawbacks of the currently known micropowders and microbeads and their methods of production.

The problem is solved by the features of the independent claims. Preferred embodiments of the present invention are provided by the dependent claims.

In one aspect, the invention relates to a micropowder comprising bacterial cellulose particles. The micropowder has an oil absorption capacity of at least 2.5 g/g and is biodegradable.

Bacterial cellulose has been found to be a surprisingly versatile and advantageous substitute for materials traditionally used to form powders as components for many industries. In contrast to metals and synthetic polymers, bacterial cellulose is highly biodegradable. Particularly in powder form, bacterial cellulose could reach a level of 75% biodegradation within 28 days. In 60 days, the bacterial cellulose powder could be completely biodegraded. The provision of the bacterial cellulose as a micropowder by washing and grinding the harvested cellulose, suspending in deionized water and oven drying, spray drying, treatment with supercritical CO₂ or freeze-drying, the micropowder could be free of organic solvents while having a large surface area to mass ratio and high porosity. The surface of the bacterial cellulose was sufficiently amphiphilic such as to be attacked and quickly broken down by microorganisms.

These results show an enormous potential for the described micropowder based on bacterial cellulose to replace metals, minerals and microplastics currently in use. Due to its rapid biodegradability in nature, there is a much lower risk of the bacterial cellulose entering the global food chain. In any case, concerns about accumulation of microplastics in bodies of water and in the food chain can be alleviated as bacterial cellulose is not only a naturally occurring material which is digestible and safely edible by many organisms, it can also be safely broken down in water, air or soil. In the environmental sense, a ground and dried bacterial cellulose micropowder which is free of organic solvents is a far more appealing alternative to the powders currently in use.

In contrast to the conventional powders, the bacterial cellulose powder was also found to be surprisingly biocompatible. In contrast to known powder additives such as talcum and aluminum, bacterial cellulose has not been linked to any health concerns. Rather, the chemically similar plant cellulose is often safely consumed by humans in the form of dietary fibre. Plant cellulose has also been used traditionally in various materials intended for contact with human skin, such as cotton fabric, bandages or traditional medicines and traditional cosmetics based on herbs, plant roots, stems, leaves and the like. It is therefore a particularly safe material.

The use of bacterial cellulose rather than plant cellulose brings further advantages. Bacterial cellulose is free from wax, lignin, pectin and hemicelluloses which are commonly found in cellulose prepared from plant sources. Being cultivated in a controlled environment, a very high degree of purity of the bacterial cellulose can be guaranteed. This is particularly useful when the inventive biodegradable powder is used in food, pharmaceutical or healthcare products.

Compared to plant cellulose, bacterial cellulose tends to have a more crystalline structure and can form characteristic ribbon-like microfibrils. In particular thin microfibrils of the bacterial cellulose are significantly smaller than those of plant cellulose, making the micropowder based on bacterial cellulose much more porous when cultured, ground and dried by a suitable technique. Advantageously, as described in more detail herein the porosity can increase the surface area and therefore the surface interactions of the bacterial cellulose particles, providing for a variety of interesting effects. Additionally, the porosity could reduce the overall density of the bacterial cellulose particles, making them especially light. The porosity was found to be influenced by the degree of crystallinity of the bacterial cellulose. As bacterial cellulose can achieve a far broader range of crystallinities, this could be adjusted in the production process of the micropowder. Crystallinities of 30 - 80 % were found to provide high porosities and surface irregularities which increased the surface area of the bacterial cellulose particles, especially when dried. This could be further improved by adjusting the average particle diameter of the micropowder, to maximize the surface area available for intermolecular interactions with surrounding media such as oil or water. The inventors have discovered that the dried bacterial cellulose particles could act as excellent thickeners and rheology modifiers due to their capacity to be suspended in large amounts in various fluid media. This enables the shelf-life of multi-phase emulsions to be lengthened, providing for high user satisfaction.

Moreover - as will be explained further herein - it is thought that the elevated porosity of the bacterial cellulose particles, especially when dried with hornification, e.g. by oven drying or spray drying, could contribute to their astonishingly high oil absorption capacities. Not only could the oil absorption of the inventive micropowder compete with that of other highly absorbent powders, it by far exceeded them. The implications of this are of crucial significance for the manufacture of a variety of consumer products. One such implication is that the microplastics, metallic and mineral powders of the state of the art can be replaced with the much safer and fully biodegradable powder of the invention, without sacrificing absorptive functionality and without reducing consumer satisfaction. Surprisingly, the functionality of the conventional powders could be drastically improved. In the case of cosmetic and personal care products, the high oil absorption of the bacterial cellulose allowed products containing the inventive powder to absorb sebum from the skin at a high capacity and over long periods of time. The products could therefore be worn comfortably for long periods of time without feelings of stickiness, greasiness or cakiness. Products could provide a very soft, matte look and texture which hides unevenness and pores. Probants also reported that such products left a pleasant, non-greasy and non-sticky after-feel.

In addition to the advantageous texture, the high oil absorption capacity of the bacterial cellulose particles allowed products to be produced with a high oil content without compromising on stability, shelf-life or aesthetics. For example, scented or flavored essential oils, cocoa butter (theobroma oil) or other vegetable oils could be carried in higher amounts in products, providing for a very high-quality impression. Potential applications include cosmetics, personal care products and foods.

Bacterial cellulose preferably comprises an ultra-fine network of cellulose fibers which are highly uniaxially oriented. This type of 3D structure can lead to a particularly high crystallinity and advantageous physico-chemical and mechanical properties, especially when manufactured into a micropowder as described herein. This structure assists in the formation of bacterial cellulose pellicles, which are preferably sheet- or film-shaped layers of bacterial cellulose. The crystallinity of bacterial cellulose can impart stiffness to the microfibrils and can be used to adjust the mechanical properties of the resulting product. These properties include flexibility, elasticity and tensile strength. The crystallinity also imparts an excellent thermal and chemical stability to the bacterial cellulose particles. Bacterial cellulose is therefore a particularly versatile material for use as a biodegradable powder.

In the sense of the invention, a "micropowder" is preferably a material comprising particles, grains or fragments. At least one spatial dimension of the particles, grains or fragments is preferably in the micro range. Preferably an average length, width, thickness or diameter of the particles, grains or fragments is less than 1000 µm. A micropowder is preferably dry, having a moisture content of less than 5 w/w%, preferably less than 1 w/w%, more preferably less than 0.5 w/w%.

In the sense of the invention, an "oil absorption capacity" is preferably a measure of the mass of oil in grams which can be bound to a single gram of the micropowder. The oil absorption capacity may be determined with reference to any oil, wherein an oil is preferably any nonpolar material composed primarily of hydrocarbons which is liquid at 25 °C. As a reference oil, sunflower oil may be used. Preferably the oil absorption capacity is determined experimentally using ASTM D281-95 or a modified variant thereof.

In the sense of the invention, "biodegradability" is preferably a measure of the length of time taken for a material to decompose after exposure to biological elements. Decomposition of cellulose preferably comprises breakage of the cellulose polymers to dimers or monomers. The biodegradability may be determined using an OxiTop measurement apparatus. A known mass of the material to be tested may exposed to microorganisms in a test bottle. The microorganisms may convert oxygen (O₂) to carbon dioxide (CO₂) during the biodegradation of the test material. OxiTop^{®} is preferably a cap-shaped pressure meter attached to a test bottle which measures biochemical oxygen demand by detecting the degree to which air pressure in the bottle decreases. After a test bottle is sealed with an OxiTop^{®} cap, the bottle may be incubated in a thermostatic chamber (e.g. IN804, Yamato Scientific, Japan). According to the OECD (Guideline for Testing Chemicals: Ready biodegradability (1992)) and the CSCL (Guideline of Chemical Substances Control Law: Biodegradability Test of Chemical Substances by Microorganisms (2018) (in Japanese*))* guidelines, when the value of biochemical oxygen demand during a test period reaches 60% of a theoretical oxygen demand for a test chemical (particularly in the 301C or the 301F test procedures), the test chemical is considered to be completely degraded (and the remaining 40% is assumed to have been assimilated by microorganisms).

In the sense of the invention, a "bacterial cellulose" is preferably a cellulose synthesized by bacteria and is also referred to as a "microbial cellulose" or "biocellulose". Bacterial cellulose is a polysaccharide chain comprising several β(1→4) linked D-glucose units. Its chemical structure can be represented by the formula (C₆H₁₀O₅)ₙ. In the sense of the invention a bacterial cellulose may also have a chemical structure deviating from said formula, in particular in the case of functionalized bacterial cellulose. The bacteria used to synthesise the bacterial cellulose may preferably belong to the genera Gluconacetobacter, Acetobacter, Aerobacter, Achromobacter, Azotobacter, Rhizobium, Lactobacillus, Agrobacterium, Pseudomonas, Alcaligenes Salmonella or Sarcina. Suitable bacteria species known to experts in the field include but are not limited to *Gluconacetobacter xylinus, Gluconacetobacter hansenii, Acetobacter xylinum, Acetobacter senegalensis, Acetobacter pasteurianum, Acetobacter rancens, Sarcina ventriculi* and *Lactobacillus Mali.* Co-cultures of two or more bacteria species may also be used. Bacterial cellulose may be cultured in a medium providing a source of carbon, optionally a source of nitrogen and any further macro- or micronutrient as deemed appropriate. Examples of carbon sources include glucose, fructose, sucrose, maltose, xylose, mannitol, glycerol and molasses. It may be preferable that a Hestrin-Schramm medium is used to culture the bacteria. Bacteria may be cultured in a static or agitated environment and culture times may vary between 30 - 300 hours. The bacterial cellulose is preferably produced in the form of one or more sheets (also referred to as pellicles, films or membranes).

In a preferred embodiment of the invention, the micropowder is biodegradable to 75 % within 35 days. Preferably, the micropowder is biodegradable to 75 % within 28 days. Unless stated otherwise, the percentage biodegradability preferably refers to a w/w percentage of the material which has been degraded or assimilated by microorganisms.

By adjusting the biodegradability of the micropowder to 75 % within 28 days, the micropowder can be rapidly biodegraded, reducing the need for any special disposal measures to prevent the micropowder from entering drains or entering the food chain. This is especially due to the small particle size, high porosity and high surface area to mass ratio of the micropowder which increases its contact with air and with microorganisms. Products formulated using the micropowder can boast a high biodegradability and environmental friendliness. The micropowder thus fills a long-standing need in the market for micropowders which can be disposed of in the same way as other consumer products without environmental concerns.

In a further preferred embodiment of the invention, the micropowder has an oil absorption capacity of at least 4 g/g, preferably at least 4.5 g/g, even more preferably at least 5 g/g. By adjusting properties of the bacterial cellulose particles to achieve said oil absorption capacities, the inventive micropowder can far outperform the known micropowders whilst being fully biodegradable and safe. Preferably the micropowder exhibits these absorption capacities for a range of oils, including but not limited to: sunflower oil, vegetable oil, canola oil, olive oil, rapeseed oil, neem oil, glycerol, lavender, tree tea oil, essential oil, jojoba oil.

Such oil capacities provided for highly sought-after properties, particularly in the field of cosmetics and personal care products. There is a high demand that products intended for use on the skin provide soft textures, especially a "blur" or "airbrush" effect. This effect can be achieved by absorbing sebum produced below the product on the skin, such that the product does not flake, crack or otherwise show discrepancies in its texture. Manufacturers of such products have competed to pack these with harmful and non-biodegradable micropowders. The inventors have discovered that the inventive micropowder could easily be configured to achieve oil absorption capacities higher than those achieved by the known micropowders.

As a mere illustration, the inventive micropowder could absorb sunflower oil at a capacity of more than 5 g/g. Under the same conditions, a micropowders made from kaolin, vinyl dimethicone crosspolymer and from PMMA (polymethylmethacrylate) could absorb sunflower oil at less than 2 g/g. The oil absorption capacity of the inventive micropowder could also by far exceed that of talcum powder, which was approximately 1.5 g/g. Probants treated with formulations comprising like-for-like quantities of the inventive micropowder and the aforementioned micropowders made from PMMA and silica reported a far greater satisfaction with the formulation comprising the inventive micropowder. In particular, a high degree of softness during wearing and a soft after-feel were reported. Probants also reported that the formulation comprising the inventive micropowder was far less greasy than the alternatives and left a much less greasy and less sticky after-feel. These results - which are explained in more detailed herein - show great potential for improving the quality of consumer products.

In further preferred embodiments of the invention, the micropowder has an oil absorption capacity of at least 7 g/g, at least 10 g/g, at least 25 g/g or more.

In a preferred embodiment of the invention the average particle diameter D₅₀ of the bacterial cellulose particles is between 1 - 1000 µm. It is however even more preferable that the average particle diameter D₅₀ of the bacterial cellulose particles is between 1 - 500 µm, preferably between 5 - 250 µm and especially preferably between 25 - 100 µm. It may also be preferred that the average particle diameter is approximately 65 µm. At these preferred average diameters, a balance could be struck between light particles and high dispersibility on the one hand and safety on the other. These particles could be very easily dispersed, filling pores, sweat glands and hiding unevenness in the skin. At the same time, the risk of crossing tissue barriers could be reduced, making the micropowder especially biocompatible.

It may also be preferred that the average particle diameter D₅₀ of the bacterial cellulose particles is between 200 - 1000 µm, preferably between 400 - 1000 µm, even more preferably between 500 - 1000 µm. At these particle sizes, the micropowder could be used in cleaning products, scrubs and exfoliants, providing the coarse texture which is desirable in these applications. At the same time, the micropowder was sufficiently light and easily dispersible. The inventive micropowder had a far greater oil absorption capacity than the known micropowders, even at these particle sizes. An excellent cleaning effect could be achieved, whilst leaving a soft after-feel on the user's skin.

In a further preferred embodiment of the invention at least 90% of the bacterial cellulose particles have a diameter of not more than 500 µm, preferably not more than 300 µm, especially preferably not more than 200 µm. Such a micropowder comprises very fine particles with a sufficiently broad particle size distribution. The broad particle size distribution can contribute to providing a semi-matte or matte finish which we may be desirable, for example in lipstick and other cosmetics.

In a further preferred embodiment of the invention, the average particle diameter D₅₀ of the bacterial cellulose particles is not more than 40%, preferably not more than 30%, even more preferably not more than 25% of the value of the 90^{th} percentile particle diameter D₉₀ of the micropowder. The micropowder can thus have a large particle size distribution. This may contribute to providing a much more matte finish in some products. It has also been found useful in providing a smooth thickening and lengthening effect in mascara liquids.

The inventors have found that the size and crystallinity of the bacterial cellulose particles constitute factors which may be advantageously used to achieve high ratios of oil absorption capacity. In this regard an average particle diameter D₅₀ of the bacterial cellulose particles of between 1 - 1000 µm and an average crystallinity of the bacterial cellulose particles of between 30 - 80% have proven particularly beneficial.

In a further preferred embodiment of the invention, an average particle diameter D₅₀ of the bacterial cellulose particles is between 1 - 1000 µm and an average crystallinity of the bacterial cellulose particles is between 30 - 80%.

At an average diameter of 1 - 1000 µm, preferably 1 - 500 µm, more preferably between 5 - 250 µm and especially preferably between 25 - 100 µm, the bacterial cellulose powder could perform a variety of functions. At these diameters, the bacterial cellulose particles could be easily dispersed in a suspension. They could thus form stable creams, lotions, paints and the like. At the same time, the bacterial cellulose powder was suitable for use in a dry product such as in loose or pressed cosmetic powders. The powder had excellent adhesion to the skin, provided a comfortable non-sticky and non-greasy texture and a matte finish. It was also found very suitable for filling pores, fine wrinkles and disguising uneven areas of skin. These properties are highly sought-after in the cosmetics sector.

At the selected range of average diameters, the bacterial cellulose powder had a very high surface area to mass ratio. This could be increased further by adjusting the crystallinity and therefore the porosity of the bacterial cellulose used. The bacterial cellulose particles could thus be made very light with a large surface area for interacting with fluids, in particular by means of intermolecular forces. The bacterial cellulose particles could thus be suspended in aqueous, inorganic and organic media without settling. Emulsions and suspensions with a long shelf-life could be produced. The high surface area to mass ratio provided by the selected range of average diameters also contributed to increasing the water and oil absorption capacity of the biodegradable powder.

The average crystallinity lying within the selected range of 30 - 80%, preferably between 40 - 80%, more preferably between 40 - 60%, especially preferably between 40 - 50 %, even more preferably around 45 % worked synergistically with the selected average particle diameters to provide optimal material properties and oil absorption capacities. At these crystallinities, the bacterial cellulose harvested from the pellicles comprised a mix of crystalline and amorphous phases. Without being limited by theory, the inventors have hypothesized that the mix of crystalline and amorphous phases led to the presence of more pores, in particular in the interfaces between phases. The bacterial cellulose material could hereby be made lighter and more porous.

The selected average degree of crystallinity also provided the bacterial cellulose with a higher stiffness, especially in comparison and to plant cellulose and to bacterial cellulose in which the crystallinity has not been adjusted or selected accordingly. When the bacterial cellulose having a crystallinity of 30 - 80 % is ground or pulverized to the selected average particle diameters of 1 - 1000 µm, the resulting particles had a range of irregular shapes, particularly polyhedral and plate-like shapes. The irregular shapes contributed to increasing the surface area to mass ratio of the particles, which increased their oil absorption capacities.

Advantageously, the irregular shapes resulting from the mix of crystalline and amorphous phases in the particles further increased the optical scattering capacities of the powders. This allowed them to serve as excellent opacifiers. When used in paints, make-up or skincare products, the optical scattering contributed to the desirable soft, matte finish.

It is to the credit of the inventors, to have discovered that the proportion of crystalline and amorphous phases in the bacterial cellulose could be finely tuned by selecting one or more suitable bacterial species and adjusting the culture conditions. The speed, frequency and duration of agitation of the culture as well as its aeration were also found to play a significant role. Combined with selected drying techniques, the degree of crystallinity could be more precisely tuned. By setting the degree of crystallinity within the defined range, the absorption and matte-effect could be optimized.

A "particle diameter" in the sense of the invention is preferably the diameter of a theoretical equivalent sphere occupying a volume equivalent to that occupied by the particle. The skilled person is aware of methods for calculating or estimating the equivalent sphere.

In the sense of the invention, an "average particle diameter" is preferably the median diameter of a plurality of particles. Unless stated otherwise, this is preferably a D₅₀ average particle diameter which is the particle diameter when the cumulative percentage of ordered particles reaches 50%. That is, a D₅₀ average particle diameter of 500 µm preferably means that 50 % of particles have a diameter greater than 500 µm and 50 % of particles have a diameter smaller than 500 µm. The D₅₀ average particle diameter may be determined by microscope image analysis, differential sieving, light scattering or otherwise. Preferably the D₅₀ average particle diameter is determined by polarization intensity differential scattering, in particular using an ULM Standard PIDS apparatus.

In a further preferred embodiment of the invention, the micropowder is produced by a method which includes grinding a bacterial cellulose pellicle and drying the ground bacterial cellulose. Initially forming bacterial cellulose particles by grinding can produce a homogenized mixture or a powder. The mixture or powder may comprise particles with irregular shapes due to the breakage caused by a grinder and with small particle sizes. The particle size may be further adjusted by drying.

In some embodiments, the drying may preferably configured to cause hornification of the bacterial cellulose.

In the sense of the invention, "hornification" is preferably a phenomenon which occurs in cellulosic material during drying or water removal, causing cellulose microfibrils to aggregate, increasing their density and strength. At the same time, hornification causes chemical groups in the cellulose chains to be blocked, improving the hydrophobicity of the produced bacterial cellulose particles. Drying methods which may induce hornification of the bacterial cellulose include oven drying and spray drying.

In further embodiments of the invention, it may be preferred that a drying method is selected to cause and/or increase hornification. An example of such a method is oven-drying. It was surprisingly found that oven-drying a fluid suspension containing the ground bacterial cellulose could increase the hydrophobicity of the bacterial cellulose particles leading to very high oil absorption capacities. The oil absorption capacities of a micropowder produced by oven-drying were higher than when freeze-drying was used.

In a further preferred embodiment of the invention, the micropowder has a surface area to mass ratio of at least 2 m²/g, preferably at least 5 m²/g, more preferably at least 10 m²/g and even more preferably at least 20 m²/g. The surface area to mass ratio may also preferably be at least 40 m²/g or at least 80 m²/g. It may be preferred that the surface area to mass ratio is not more than 100 m²/g in order to limit flammability and reactivity.

The surface area to mass ratio not only increased dispersibility, by increasing the intermolecular interactions between the micropowder and a medium in which it is carried, but also improved the oil and water absorption capacities. In addition, the surface area to mass ratio increases the capacity of the bacterial cellulose particles to adsorb and desorb molecules and living cells. The biodegradation of the micropowder could hereby be accelerated. Tuning of the surface area to mass ratio could be carried out by setting the speed and/or duration of a grinding step of the bacterial cellulose.

Additionally, the surface area to mass ratio could be increased by subjecting the bacterial cellulose to processes which cause an increase in the porosity of its material. The inventors have discovered that an increase in through pores and blind pores in the bacterial cellulose particles could significantly raise the surface area to mass ratio and in turn the oil absorption capacity. Examples of processes which were found to increase the porosity were the culture process itself, in particular where agitation was applied to the culture medium. By examining the effect of different compositions of the culture medium on the morphology of the synthesized cellulose using SEM scanning microscopy, it has been showed that the nutrients contained in the microbiological substrate can also affect the degree of porosity, which results from the density of the cellulose fiber network *(*Molina-Ramirez, C.; Castro, C.; Zuluaga, R.; Gañán, P. Physical Characterization of Bacterial Cellulose Produced by Komagataeibacter medellinensis Using Food Supply Chain Waste and Agricultural By-Products as Alternative Low-Cost Feedstocks. J. Polym. Environ. 2018, 26, 830-837*).* Moreover, the porosity could be increased by adjusting post-treatment process such as the drying process (Tang, W.; Jia, S.; Jia, Y.; Yang, H. The influence of fermentation conditions and post-treatment methods on porosity of bacterial cellulose membrane. World J. Microbiol. Biotechnol. 2010, 26, 125-13131*).* For example, it was found that spray-drying or oven-drying could increase the number of pores in the bacterial cellulose.

In a further preferred embodiment of the invention an average crystallinity of the bacterial cellulose particles is between 40 - 80%, preferably between 40 - 60%, especially preferably between 40 - 50%. It was surprisingly found that bacterial cellulose particles having such crystallinities were particularly light and easily dispersible. At the same time, the particles had a high porosity and large specific surface area. The resulting micropowder achieved very high water and oil absorption capacities.

Bacterial cellulose produced under static cultivation conditions usually takes the form of one or more hydrous pellicles comprising an interconnected network of fibrils assembled in numerous dense layers. Cellulose crystallinity and the mechanical properties that crystallinity provides to cellulose are major factors influencing the layered bacterial cellulose structure formed during biosynthesis *(Ruan et al., 2016).* There are various key parameters of the bacterial fermentation process that can be used to control the structural features of the obtained cellulose fibres. The degree of crystallinity can depend on:
(i) the chosen method of culturing cellulose-synthesizing micro-organisms *(*Czaja, W.; Romanovicz, D.; Brown, R.M. Structural investigations of microbial cellulose produced in stationary and agitated culture. Cellulose 2004, 11, 403-411*),*
(ii) the types of carbon source and other components of the medium (Yim, S.M.; Song, J.E.; Kim, H.R. Production and characterization of bacterial cellulose fabrics by nitrogen sources of tea and carbon sources of sugar. Process Biochem. 2017, 59, 26-3), wherein a Hestrin-Schramm medium was found suitable for providing a crystallinity between 30 - 80%, and
(iii) the method of drying used (Stanislawska, A.; Staroszczyk, H.; Szkodo, M. The effect of dehydration/rehydration of bacterial nanocellulose on its tensile strength and physicochemical properties. Carbohydr. Polym. 2020, 236, 1160235*).* The inventors have found that in the case of oven-based or spray drying, the degree of crystallinity of bacterial cellulose was slightly higher than during drying by lyophilization or freeze drying. The above crystallinities may thus be achieved by adjusting these factors, wherein oven or spray drying are especially preferred.

The degree of crystallinity of bacterial cellulose is correlated with its degree of polymerization. In a preferred embodiment of the invention, the bacterial cellulose has a degree of polymerization between 1000 - 20000. As a comparison, plant cellulose has degrees of polymerization between 10 - 100 making it less versatile in its range of physical properties. Without wishing to be bound to any particular theory, it is thought that a higher degree of polymerization, i.e. the production of longer polymer chains, leads said chains to be more tightly packed in the culture and to crystallize more easily. On the other hand, when the culture is subjected to stirring or agitation, it is thought that the length and packing of said polymer chains is limited by the shear forces present in the fluid medium. A lower degree of polymerization and a lower degree of crystallization results.

In the sense of the invention, a "degree of polymerization" is preferably an average number of monomeric units in the chains of a polymer.

The degree of polymerization has also been linked to the porosity of the bacterial cellulose particles. During the polymerization process of bacterial cellulose, various types of pores may be formed. These include through pores, blind pores and closed pores.

In the sense of the invention, a "through pore" is preferably a channel extending through a particle, wherein the channel is open at two distinct ends. That is, a through pore is preferably accessible from two distinct openings in the surface of a bacterial cellulose particle.

In the sense of the invention, a "blind pore" is preferably a channel extending at least partially through a particle, wherein the channel is open at only one end. That is, a blind pore is preferably accessible only from a single opening in the surface of a bacterial cellulose particle.

In the sense of the invention, a "closed pore" is preferably a channel which is encapsulated within the bacterial cellulose particle and is not accessible from any of its surfaces.

Through pores make a significant contribution to the permeability and therefore to the water and oil absorption capacities of the bacterial cellulose micropowder. A large number of through pores in the bacterial cellulose particles can also increase the surface area to volume ratio and the surface are to mass ratio of the particles, further increasing their absorption capacities.

Blind pores also contribute to increasing the surface area to volume ratio and the surface area to mass ratio of the particles. Both through pores and blind pores could thus contribute to the absorption, adsorption and desorption capacities of the micropowder. The biodegradability of the micropowder could also be improved by the presence of such pores.

Through pores and blind pores were found to be particularly advantageous in the absorption of sebum from the skin. Hetero atoms forming functional groups present in the cellulose (e.g. oxygen, carbonyl groups, sulfur, phosphorus or nitrogen) are fairly electronegative and possess attractive power to long chain fatty acids, esters, proteins, lipids, carbohydrates or drug molecules. Molecular dynamic simulation studies have confirmed that dry bacterial cellulose surfaces have a high affinity for oils with ΔG_{bind} values of -300 kcal/mol being calculated for vegetable oils. Sebum in particular - comprising inter alia esters and fatty acids - can be attracted and absorbed very well into these bacterial cellulose pores. A greater number of pores or a large size distribution can improve the absorption capacities and the fluid flow there through (Burggraaf, A. J. & Cot, L. Fundamentals of inorganic membrane science and technology. 4, (Elsevier, 1996), Joghataei, M., Semnani, D., Salimpour, M. R., Ashrafi, Z. & Khoeini, D. Comparison of heat transfer coefficient for different fabrics by vapor-compression system. Int. J. Eng. Technol. 5, 11-15 (2016*)).*

Although closed pores do not directly contribute to the transport of mass through the nondegraded particles, they can improve the strength and pliability of the bacterial cellulose while reducing the density.

It may be preferable that the bacterial cellulose particles have a high percentage of through pores. It may be preferable that at least 20 %, at least 30 % or at least 40 % of the void volume of the particles is located in through pores.

It may also be preferable that the bacterial cellulose particles have a high total percentage of through pores and blind pores. It may be preferable that at least 50 %, at least 60 % or at least 70 % of the void volume of the particles is located in blind pores and through pores. This may improve the absorptive and adsorptive capacities of the micropowder.

In a further preferred embodiment of the invention, the bacterial cellulose particles have a porosity of at least 3 %, preferably at least 5 %, more preferably at least 10 %, even more preferably at least 20 %.

In a further preferred embodiment of the invention, the micropowder has a porosity of at least 20 %, preferably at least 30 %, more preferably at least 40 % and even more preferably at least 50 %.

In the sense of the invention, "porosity" of a particle is preferably a proportion of the volume of the particle which is a void. The "porosity" of a material such as a micropowder, is preferably a proportion of the volume of the material which is a void. The porosity is preferably given as a percentage. The percentage porosity may be calculated using the formula %Porosity = V_{V}/V_{T} x 100, wherein V_{V} is the void volume and V_{T} is the total volume.

The percentage porosity and the distribution of pore types in the bacterial cellulose depends on the density of the cellulose fiber network. It was found that the cellulose fiber network density depends on the bacterial culture conditions and the drying technique used (Tang, W.; Jia, S.; Jia, Y.; Yang, H. The influence of fermentation conditions and post-treatment methods on porosity of bacterial cellulose membrane. World J. Microbiol. Biotechnol. 2010, 26, 125-13131). Examining the morphology of bacterial cellulose cultured in different media compositions using SEM scanning microscopy showed that the nutrients contained in the medium affect the degree of porosity (Molina-Ramirez, C.; Castro, C.; Zuluaga, R.; Gañán, P. Physical Characterization of Bacterial Cellulose Produced by Komagataeibacter medellinensis Using Food Supply Chain Waste and Agricultural By-Products as Alternative Low-Cost Feedstocks. J. Polym. Environ. 2018, 26, 830-837*).* The porosity could also be affected by the agitation and aeration of the medium.

Different drying techniques were also found to affect the porosity of the bacterial cellulose particles *(*Bruno Thibault, Cristina Ratti, Seddik Khalloufi A mathematical tool for estimating the efficiency of pore formation during dehydration, Journal of Food Engineering, Vol 323, 2022, 110981*).* For example, it has been found that decreasing the pressure in a freeze-drying process could increase the porosity of many organic materials (Vasiliki P. Oikonomopoulou, Magdalini K. Krokida, Vaios T. Karathanos, The influence of freeze drying conditions on microstructural changes of food products, Procedia Food Science, Volume 1, 2011, Pages 647-654).

In a further preferred embodiment of the invention, the bacterial cellulose particles are mesoporous with an average pore radius of 1 - 50 nm, preferably 10 - 30 nm, more preferably 5 - 20 nm, even more preferably around 7 nm. Preferably, the surface area to mass ratio of the bacterial cellulose particles is 10 - 100 m²/g, more preferably 10 - 80 m²/g.

Surface area is preferably the interface through which a solid interacts with its surroundings, especially liquids and gases. Surface area can be created by particle size reduction, e.g., grinding and milling as well as making materials porous. The surface area of a solid material is typically determined by physical adsorption of a gas on the surface of the solid and by calculating the amount of adsorbate gas corresponding to a monomolecular layer on the surface. The results of such an analysis are set out further herein.

In a further preferred embodiment of the invention, a surface of the bacterial cellulose particles displays filamentous protrusions similar to microvilli. Such microvilli-like protrusions were observed in bacterial cellulose particles produced by the preferred methods set out herein.

Without wishing to be limited by theory, it is thought that these microvilli-like protrusions improve the oil and water absorption capacities of the bacterial cellulose particles by increasing the surface area to mass ratio. Especially in dry form, molecular dynamics simulations have shown that bacterial cellulose fibers have a high affinity towards saturated hydrocarbon molecules and will be published elsewhere. For common oils such as glycerol and vegetable oil, free binding energies of ΔG_{bind} between -50 to -300 kcal/mol were calculated. It is thought that this property of the bacterial cellulose microfibers could be harnessed to dramatically improve the oil absorption of a micropowder by exposing such microprotrusions on the surface of the bacterial cellulose particles.

In the sense of the invention "microvilli-like protrusions" are preferably elongate projections from the surface of a bulk material, preferably comprising fibers aligned approximately parallel to the plane formed by the surface of the bulk material. The protrusions may for instance exhibit diameters of considerably less than 1 µm diameter, preferably less than 100 nm with an aspect ratio of more than 5, preferably more than 10.

In the sense of the invention, a "particle" is preferably a discrete portion of material having a well-defined boundary in all directions. Preferably a particle has a mass of less than 1 gram and/ or a maximum dimension of up to 5 mm, preferably up to 1000 µm. A particle is preferably distinguished from a fiber in that its aspect ratio is less than 50:1, more preferably less than 20:1, even more preferably less than 10:1. In a further preferred embodiment of the invention the bacterial cellulose particles have an average aspect ratio less than 10:1, preferably less than 5:1, more preferably less than 2:1.

In the sense of the invention, an "aspect ratio" is preferably a ratio of its maximum dimension to its shortest dimension. It may be preferably for the aspect ratio to be a Feret aspect ratio determined in terms of Feret diameters, in particular for polyhedral, globular, disc-shaped, plate-shaped, ellipsoidal or spherical particles. Preferably the Feret aspect ratio is a ratio of the maximum to the minimum Feret diameter of a particle. The maximum Feret diameter is preferably the greatest distance between two parallel tangents of the particle at any possible angle. The minimum Feret diameter is preferably the smallest distance between two parallel tangents of the particle at any possible angle. The maximum and minimum Feret diameters may be determined microscopically and/or using a computer-based analysis software.

At lower aspect ratios, the bacterial cellulose particles are preferably closer to being spherical. Such particles were found less likely to agglomerate and form networks than needle-shaped or rod-like particles. As such, the micropowder was more stable and had a longer shelf-life, despite the presence of low amounts of residual moisture. When the bacterial cellulose particles having low aspect ratios were suspended, stable suspensions could be formed without unwanted colloidal gel formation. Ease of spreadability of creams or lotions could thus be maintained.

The aspect ratio of the particles also affected their optical scattering properties. It was found that low aspect ratios could scatter light to a lesser extent, such that products could leave a more shiny appearance. This was observed especially in micropowders having spherical particles.

In some preferred embodiments of the invention, the bacterial cellulose particles are polyhedral in shape. Polyhedral particles were found to be more light-scattering than spherical particles, such that a consumer product comprising the micropowder of this embodiment could leave a more matte finish. In addition, polyhedral particles had a larger surface area to mass ratio than spherical particles. This contributed further to the absorption capacities of the micropowder.

In the sense of the invention, a "polyhedral" particle shape is preferably a three-dimensional shape having multiple flat surfaces, wherein adjacent flat surfaces are preferably at an angle to one another. Preferably the polyhedral particle has at least six flat faces, more preferably at least eight. It may be preferable that the polyhedral particle does not have more than 30, preferably not more than 20 flat faces.

In some preferred embodiments of the invention, the bacterial cellulose particles are plate-shaped. Plate-shaped particles could be packed very tightly and could therefore be used to formulate products with a high coverage. This may be particularly advantageous in paints and cosmetics.

In the sense of the invention, the term "plate-shaped" preferably refers to a particle having two flat faces, wherein the two flat faces are preferably approximately parallel to one another.

In the sense of the invention, a micropowder in which particles have a particular shape, is preferably a micropowder in which at least 50%, more preferably at least 60%, 70% or more of the particles have the said shape.

In a further preferred embodiment of the invention the bacterial cellulose micropowder is free of organic solvents. The micropowder could hereby be made highly biocompatible and suitable for use on the skin. As cellulose fibres are somewhat hydrophobic, volatile organic solvents such as ethanol and acetone are sometimes used as carriers during the processing of the cellulose. By eliminating or avoiding these components, a much more skin-friendly micropowder could be produced without risk of irritation. The production process of the micropowder could also be made safer and more environmentally friendly.

Preferably the micropowder has a moisture content of less than 10 w/w%, preferably less than 7 w/w% and especially preferably less than 5 w/w%. Even more preferably the moisture content is less than 0.5 w/w%. These low moisture contents provided for a higher stability and shelf-life of the micropowder, preventing unwanted aggregation of particles. Additionally, the low moisture contents improved the absorption capacities of the micropowder, especially the oil absorption capacity. As is well-known, water and oil tend to be immiscible. A reduction in water content improved the contact between the micropowder and oil. As the pores and outer surfaces of the bacterial cellulose particles were free of adsorbed water, they could instead attract oil particles and hold them by means of intermolecular forces. The resulting micropowder thus exhibited a very high absorption performance.

In a further preferred embodiment of the invention, the bacterial cellulose particles are nonfunctionalised. Non-functional bacterial cellulose particles are naturally somewhat hydrophobic, such that they could attract the oily components released from skin. Nonfunctionalised bacterial cellulose particles were also found particularly effective for use as a micropowder in cleaning products, toothpaste and the like, due to their affinity for oils and proteins. Moreover, such a micropowder needs to undergo fewer chemical processing steps and can boast the status of being a natural product.

In a further preferred embodiment of the invention the bacterial cellulose particles comprise functionalized cellulose, wherein the functionalized cellulose is preferably functionalized by addition of an alkyl group, an acyl group, an ester, an amine, a carboxyl group, a carboxymethyl group, phosphorus and/or sulphur, preferably at the C6 position. Functionalisation of the bacterial cellulose particles can lend specific properties to the micropowder such as hydrophobicity, polarity, amorphousness and solubility in different media. For example, carboxylation at C6 could increase the polarity of the bacterial cellulose and lend it partial solubility in aqueous media. The functionalized micropowder could thus act as a good rheology modifier in liquid products, lending a desirable shear-thinning effect. Shear-thinning is especially useful in products which are to be easily spread over a surface but may remain stable thereafter. Such products include wall paints, mayonnaise, ketchup and skin creams.

In a further preferred embodiment of the invention, the micropowder is configured as a pigment. It may be preferred that the bacterial cellulose particles of the micropowder are coated with a dye. It may be preferred that the bacterial cellulose particles are functionalized before being coated with the dye. For example, the bacterial cellulose particles may be carboxylated such that their surface has a negative surface charge. The bacterial cellulose particles may then be coated by adsorption of molecules of a dye having a positive surface charge. Alternatively, the bacterial cellulose particles may be functionalized such that these have a positive surface charge before being coated by a dye having a negative surface charge. The dye may preferably be an organic dye. Such a micropowder could not only improve the texture and feel of a consumer product but also finely adjust its tint. Particularly in cosmetic products such as foundation creams, a close match between the color of the product and the user's skin is highly desirable. Such a micropowder can provide this color-matching function whilst remaining safe and biodegradable.

In a further preferred embodiment of the invention, the micropowder is configured as a carrier for a pharmacologically active molecule. For example, the pharmacologically active molecule may comprise a drug molecule, urea, a lipid or a protein. Preferably said pharmacologically active molecule is adsorbed onto the surface of the bacterial cellulose particles. Such a micropowder can be used in particularly safe and biocompatible medical products such as medicated ointments.

In a further aspect, the invention relates to a biodegradable powder comprising bacterial cellulose particles, wherein an average particle diameter D₅₀ of the bacterial cellulose particles is between 1 - 1000 µm, preferably between 1 - 500 µm, more preferably between 5 - 250 µm and even more preferably between 25 - 100 µm, and an average crystallinity of the bacterial cellulose particles is between 30 - 80%, preferably between 40 - 60 %. It was surprisingly found that at this combination of parameters, the bacterial cellulose particles had a very high surface area to mass ratio, a high porosity and a very high oil absorption capacity. For example, an oil absorption capacity of more than 5 g/g of sunflower oil at 25 °C could be achieved.

In a second aspect, the invention relates to a method for the manufacture of a micropowder. The method comprises the following steps:
- producing bacterial cellulose from a bacterial culture such that an average crystallinity of the bacterial cellulose produced is between 30 - 80%,
- grinding bacterial cellulose to form bacterial cellulose particles with an average particle diameter D₅₀ of 1 - 1000 µm, and
- drying the ground bacterial cellulose particles.

By producing the bacterial from a culture configured to give the bacterial cellulose an average crystallinity of 30 - 80%, the bacterial cellulose bulk material comprises an adequate mix of crystalline and amorphous phases. Crystalline phases preferably comprise ordered cellulose chains which are preferably tightly packed parallel to each other and bound by hydrogen bonds. The cellulose chains of the amorphous phases however are preferably disordered and bound by weaker intermolecular forces. The hydrogen bonds make the crystalline phases much stiffer than the amorphous phases, such that their breakage preferably leads to the formation of flat rather than spherical or ellipsoid surfaces. The result preferably comprises polyhedral or plate-like particles. These provide a large surface area to mass ratio and are highly optically scattering, allowing the micropowder to provide a non-greasy feel and a matte look. At the same time, the mix of crystalline and amorphous phases can reduce the tightness of the packing of the cellulose microfibrils. This can lead to a higher porosity in the bacterial cellulose, increasing oil absorption capacity.

By grinding the bacterial cellulose to an average particle diameter of 1 - 1000 µm, a powder or suspension of bacterial cellulose particles having a very high surface area to mass ratio can be provided. The particles are thus very dispersible, making stable suspensions. Due to the elevated surface area to volume ratio, the particles are also highly oil- and water-absorbent.

Drying the ground bacterial cellulose particles can release fluids from their pores and increase the porosity further. The drying step further adjusts the shape, morphology and surface energy of the bacterial cellulose particles. During the drying process the micropowder may preferably attain a shape that can vary from approximately spherical to globular to deformed disc shapes. Globular to deformed disc shapes scatter light differently than do spheres, and the optical scattering that results in different outcomes some cosmetic applications, e.g., shiny appearance.

The drying step was found to alter the surface energy of the bacterial cellulose particles in ways that can improve formulations and sensorial experience. Generally, the surfaces of micropowders and microbeads must be modified in order to make them compatible with various cosmetic formulations. To aid cosmetic formulation, provide functional properties and enhance the aesthetic experience, known microbeads of starch and silica are usually subjected to various kinds of surface treatments. It was surprisingly found that drying the ground bacterial cellulose could improve their hydrophobicity and their affinity to oils, making them suitable for use in emulsions and oil-based creams. The micropowders also had an astonishingly high oil absorption capacity in comparison to known micropowders.

Furthermore, drying of the ground bacterial cellulose provides a micropowder which is free of organic solvents, has a low moisture content and a long shelf-life.

Preferably, the drying step occurs under sterile conditions.

In the sense of the invention, an average "crystallinity" of a material is preferably a percentage of the total volume of the material that is crystalline.

In the sense of the invention, a "grinding" step is preferably a process of breaking a bulk material to particles and is also referred to herein as crushing, milling, pulverization or homogenization. As a mere example, grinding may be carried out in a colloid mill.

In the sense of the invention, a "drying" step is preferably a process of separating water from a solution, suspension or a moisture-containing solid by applying temperature and/or pressure changes. Preferably the temperature changes comprise boiling, freezing and/or sublimation of the water. Preferably the pressure changes comprise reducing the pressure in a system, in particular by using a vacuum to lower the boiling or sublimation point of the water.

In a preferred embodiment of the invention, the bacterial culture is subjected to agitation and/or stirring during the step of producing bacterial cellulose from the bacterial cultures. Agitating the bacterial cellulose culture has been found to limit the crystallinity and could thus be used to adjust the balance of crystalline and amorphous phases. Agitation may preferably be carried out at 5 - 250 rpm (rotations per minute), more preferably 100 - 200 rpm.

In a preferred embodiment of the invention, the bacterial cellulose is washed before grinding. Washing may be carried out using deionized water.

In a further preferred embodiment of the invention the bacterial cellulose is at least partially dried between production and grinding, wherein partial drying preferably occurs in an atmosphere having a relative humidity of up to 85 %, preferably up to 80 %, and a temperature between 20 - 40 °C, preferably 25 - 35 °C, for a period of 3 - 10 days, preferably for a period of 5 - 9 days.

By at least partially drying the bacterial cellulose, the culture process could be ended, and the bacterial cellulose could be ground without the culture medium. The efficiency of the grinding process could be improved.

In a further preferred embodiment of the invention the bacterial cellulose is treated with a liquified gas, preferably liquid nitrogen, before grinding. The treatment with a liquified gas could rapidly reduce the temperature of the bacterial cellulose, increasing its brittleness and creating cracks in the crystalline structure which are thought to increase the porosity and the irregularity of the resulting bacterial cellulose particles. In particular when grinding occurred within 30 minutes after treatment with the liquefied gas, the bacterial cellulose particles had a polyhedral shape which is advantageous for increasing the surface-area to mass ratio of the micropowder.

It may be preferable that the grinding step comprises multiple grinding cycles. The multiple grinding cycles may correspond to different degrees of coarseness, such that the bacterial cellulose particle sizes are reduced in steps. This may enable use of different grinders which are specialized for producing different particle sizes, improving the efficiency of the production process. The multiple grinding cycles may preferably be separated by treatments with liquefied gas.

In a further preferred embodiment of the invention the ground bacterial cellulose particles are dried by means of freeze-drying, oven drying, spray drying, treatment with supercritical CO₂ or a combination thereof. These drying methods were found particularly suitable for achieving low moisture levels in the bacterial cellulose and leaving the bacterial cellulose particles with an adequate surface energy, such that they were highly oil absorbent.

In a further preferred embodiment of the invention before drying, the ground bacterial cellulose particles are mixed with deionised water to form a suspension having a concentration of 0.5 - 5 w/w% of bacterial cellulose in water, more preferably having a concentration of 1.0 - 3.5 w/w%.

By suspending the ground bacterial cellulose in water, a suspension could be formed with a high degree of chemical purity and without the use of organic solvents. The resulting micropowder could be made especially safe and suitable for use on sensitive skin and in pharmaceutical products. Moreover, the absence of trace salts and the like in the deionized water enabled maintaining the low polarity and high oil-affinity of the micropowder. At the selected concentrations, the suspension was stable and agglomeration of the suspended particles could be avoided. The suspension was therefore particularly suitable for being atomized.

In a further preferred embodiment of the invention, the suspension of bacterial cellulose in water is spray dried, wherein the spray drying preferably comprises the steps of:
- atomising the suspension of bacterial cellulose in water to form droplets suspended in a gas, wherein the atomisation is preferably aided by use of a pressurised gas, in particular air or an inert gas, especially preferably nitrogen,
- subjecting the suspended droplets to a flow of heated air, such that the water content of the droplets is reduced, the heated air preferably having a temperature between 80 - 250 °C, especially preferably 150- 190 °C,
- collecting the dried particles.

Spray drying the suspension provided fine particles with a controlled particle size distribution and a high degree of purity. Hornification which occurred during the drying process further improved the chemical stability of the micropowder and its hydrophobicity, whilst keeping interstices in the bacterial cellulose open for absorption and adsorption.

The spray drying preferably occurs in a drying chamber. In some preferred embodiments of the invention, the spray drying was carried out by pumping the suspension into the drying chamber, pumping heated air at a volumetric flow rate of 500 - 700 NUh (Normal Litres per hour) into the drying chamber and aspirating the air out of the drying chamber.

The heated air entering the drying chamber preferably has a relative humidity of up to 30 %, more preferably up to 20 %. The aspirated air preferably has a relative humidity of up 35 % or more. The air flow causes the evaporation of the water from the falling droplets such that solid particles are formed during their downward trajectory through the drying chamber.

In a further preferred embodiment of the invention, the atomization of the suspension of bacterial cellulose in water occurs with the help of a pressurized gas, in particular nitrogen gas. Using such an atomization, very fine particles of average diameter as low as 300 nm could be produced.

In a further preferred embodiment of the invention, collection of the dried particles occurs by means of an electrostatic particle separator. Depending on the functional groups present at its surface, the surface charge of the bacterial cellulose particles may be positive or negative. The magnitude of the charge may depend on the particle size. An electrostatic particle separator can be used to collect particles having a pre-determined range of sizes, such that the micropowder has a finely controlled particle size distribution. The electrostatic particle separator could also be used to improve the yield of the drying process. Yields of up to 90 % were achieved when collecting particles by electrostatic particle separation, in comparison to yields of 60 - 70 % when conventional methods were used.

In a further preferred embodiment of the invention, the suspension of bacterial cellulose in water is oven dried. The suspension is preferably placed in an oven dryer with hot air circulation at 80 - 160 °C, preferably 90 - 120 °C for a period of 12 - 72 hours, preferably 20 - 28 hours.

In a further preferred embodiment of the invention the suspension of bacterial cellulose in water is freeze-dried, wherein the freeze-drying preferably comprises the steps of:
- freezing the suspension at a temperature between -10 and -50 °C, preferably between -15 and -25 °C,
- reducing the pressure of a controlled atmosphere surrounding the frozen suspension to less than 50 mbar, preferably less than 1 mbar, more preferably less than 0.5 mbar, even more preferably less than 0.2 mbar, such that ice from the frozen suspension is sublimated and a resulting cake has a moisture content of less than 7 w/w%, preferably less than 5 w/w% and especially preferably less than 1 w/w%.

Freeze-drying the suspension of bacterial cellulose in water was found to increase porosity and reduce crystallinity. It was therefore an advantageous method for adjusting the material properties of the bacterial cellulose particles after culturing.

In a further preferred embodiment of the invention, the cake resulting from the freeze-drying process is subjected to a further grinding step. The grinding step preferably comprising crushing the cake to a micropowder, in particular using a blend-cutter.

In a further preferred embodiment of the invention the dried bacterial cellulose particles are separated by a particle separation module according to their particle size, such that the resulting micropowder comprises bacterial cellulose particles having an average diameter D₅₀ between 1 - 500 µm, preferably between 5 - 250 µm and especially preferably between 25 - 100 µm, and at least 90% of the bacterial cellulose particles have a diameter of not more than 500 µm, preferably not more than 300 µm, especially preferably not more than 200 µm.

Using a particle separation module, the average diameter and the size distribution of the micropowder can be finely adjusted. At the selected particle diameters, the micropowder displayed highly sought-after absorption properties. These are considered to be at least partially due to the high surface area to mass ratio of a micropowder having the selected particle diameters. The texture and coverage of the micropowder were also found to provide advantageous qualities, especially in the cosmetics industry.

In a further preferred embodiment of the invention the particle separation module comprises
- one or more sieve-shakers,
- an inertial separator, in particular a sedimenter,
- a centrifugal separator, in particular a cyclone,
- an electrostatic separator
or a combination thereof.

In a further preferred embodiment of the invention, all equipment is sterilized before use to produce the micropowder.

The micropowder of the invention may preferably be used in a consumer product composition, in particular in a cosmetic, personal care, food product, cleaning product, paint or coating. Preferred examples of cosmetic products and personal care products in which the micropowder may be used include a cream, emulsion, foam, gel, lotion, milk, mousse, solution, stick, ointment, paste, powder (loose or pressed), cream-to-cosmetic, spray, or suspension. A cosmetic product may preferably be any colored cosmetic used on the skin, hair, eyes, or lips, such as concealing sticks, foundation (wet or dry), stage make-up, mascara (cake or cream), eye shadow (liquid, pomade, powder, stick, pressed or cream), hair color, lipsticks, lip gloss, kohl pencils, eye liners, blushers, eyebrow pencils, and cream powders. Other exemplary cosmetic compositions include, nail enamel, skin glosser stick, hair sprays, face powder, legmakeup, insect repellent lotion, nail enamel remover, perfume lotion, and shampoos of all types (gel or liquid). In addition, the micropowder may be used in shaving cream (concentrate for aerosol, brushless, lathering), hair grooming, cologne stick, cologne, cologne emollient, bubble bath, body lotion (moisturizing, cleansing, analgesic, astringent), after-shave lotion, after-bath milk and sunscreen lotion.

In a further aspect, the invention relates to a cosmetic or personal care product comprising the micropowder of the invention. The cosmetic or personal care product is preferably a skin care product, including for example an anti-ageing product, a treatment product for oily and acne-prone skin , a face mask, a moisturizer, a buttercream or a lotion, a colored cosmetic product, including for example a foundation, a concealer, a loose or pressed powder, a mascara, a lipstick or a lip gloss, a hair care product, including for example a fluid shampoo, a dry shampoo, a shampoo bar, a hair mask, a hair conditioner or a hair styling product or a toiletries product including for example an anti-perspirant, a deodorant or an oral care product.

In the case of a cosmetic or personal care product, it may be preferable that the micropowder be present in a formulation at a concentration of 0.5 - 50 w/w %, more preferably 0.5 - 30 w/w %.

Advantageously, the inventive micropowder may be used in different standard formulations for cosmetic or personal care products in order to improve the product properties. In particular, the inventive micropowder may be used to replace known absorbing powders, texturizing agents or sensory enhancers, such as synthetic polymer or mineral powders, yet conveying substantially improved properties. The inventive micropowder may be preferably used to replace the known absorbing powders of known formulations in similar or smaller amounts.

In a preferred embodiment of the invention, the cosmetic or personal care product comprises a fluid formulation comprising 1 - 50 w/w % of the micropowder, 1 - 90 w/w % of a solvent, 1 - 10 w/w % of a humectant, 0.1 - 10 w/w % of a rheology modifier, 0.1 - 30 w/w % of an emollient and 0.1 - 30 w/w % of an emulsifier. It may be preferable that the formulation further comprises an anti-oxidant, a preservative, a fragrance, a pigment, a structuring agent, a pH-adjuster, a stabilizer, a binder, a filler and/or a surfactant.

In a further preferred embodiment of the invention, the cosmetic or personal care product comprises a solid formulation which in turn comprises 1 - 50 w/w% of the micropowder, 1 - 50 w/w % of a binder and 1 - 90 w/w % of a filler. Optionally, the solid formulation may further comprise a pigment, a fragrance, a surfactant, a conditioner agent, an emollient, a structuring agent, a lake-dispersion agent, a pearling agent, an anti-oxidant and/or a preservative. In a further preferred embodiment of the invention, the cosmetic or personal care product is a lotion for use on the body or face. The lotion preferably comprises
- 55 - 85 w/w % of a solvent,
- 1-10 w/w % of a humectant,
- 0.1 - 1 w/w % of a thickener,
- 0.5 - 5 w/w % of the inventive micropowder,
- 2 - 20 w/w % of an emollient, in particular comprising one or more oils,
- 1-10 w/w % of an emulsifier,
- 0.1 - 3 w/w % of an antioxidant, in particular tocopherol,
- 0.1 - 3 w/w % of a preservative, in particular EUXYL PE 9010, and
- 0.1 - 3 w/w % of a fragrance, in particular an essential oil,
wherein the w/w % fractions relate to the total weight of the lotion and the sum of the w/w % fractions is less or equal to 100 %.

In a further preferred embodiment of the invention, the cosmetic or personal care product is a lotion for use on the body or face. The lotion preferably comprises approximately
- 76 w/w % of a solvent, in particular water,
- 5 w/w % of a humectant, in particular glycerine,
- 0.2 w/w % of a thickener, in particular xanthan gum,
- 1.5 w/w % of the inventive micropowder,
- 10 w/w % of an emollient, in particular comprising one or more oils,
- 5.5 w/w % of an emulsifier,
- 0.5 w/w % of an antioxidant, in particular tocopherol,
- 1 w/w % of a preservative, in particular EUXYL PE 9010, and
- 0.25 w/w % of a fragrance, in particular an essential oil.

The lotion preferably has a pH of approximately 6.9. Preferably the lotion has a viscosity of approximately 24,600 cP, in particular 24 hours after production of the lotion and measured at 26 °C. The viscosity of the inventive lotion was significantly higher than that of a lotion having the same formulation but using a synthetic petroleum-based polymer micropowder (17,866 cP) and a silica-based micropowder (18,567 cP) in place of the inventive micropowder.

In a further preferred embodiment of the invention, the cosmetic or personal care product is a mascara. The mascara preferably comprises
- 1 - 7 w/w % of a humectant,
- 0.1 - 3 w/w % of a rheology modifier,
- 30 - 80 w/w % of a solvent,
- 3 - 30 w/w % of a pigment,
- 2 - 20 w/w % of a structuring agent,
- 2 - 20 w/w % of an emulsifier,
- 0.5 - 10 w/w % of the inventive micropowder,
- 0.5 - 3 w/w % of a preservative,
- 0.01 - 3 w/w % of an antioxidant, and optionally
- a pH adjuster, in particular sodium hydroxide,
wherein the w/w % fractions relate to the total weight of the lotion and the sum of the w/w % fractions is less or equal to 100 %.

In a further preferred embodiment of the invention, the cosmetic or personal care product is a mascara. The mascara preferably comprises approximately
- 3 w/w % of a humectant, in particular propylene glycol,
- 0.3 w/w % of a rheology modifier, in particular xanthan gum,
- 60 w/w % of a solvent, in particular water,
- 9 w/w % of a pigment, in particular a black pigment,
- 12 w/w % of a structuring agent, in particular comprising 8 w/w % beeswax and 4 w/w% carnauba wax with respect to the final product,
- 8 w/w % of an emulsifier, in particular comprising 3 w/w % cetearyl alcohol and 5 w/w % stearic acid with respect to the final product,
- 2 w/w % of the inventive micropowder,
- 1 w/w % of a preservative, in particular EUXYL PE 9010,
- 0.05 w/w % of an antioxidant, in particular tocopherol, and optionally
- a pH adjuster, in particular sodium hydroxide.

The mascara preferably has a pH of approximately 7 and a viscosity of approximately 434,000 cP, in particular 24 hours after production of the mascara and measured at 25 °C. It was found that the micropowder could significantly improve the volume-increasing and lengthening effect of the mascara. It is thought that this is at least partially due to a broad particle size distribution.

In a further preferred embodiment of the invention, the cosmetic or personal care product is a loose powder. The loose powder preferably comprises
- 5 - 40 w/w % of a pigment,
- 1 - 50 w/w % of the inventive micropowder,
- 5 - 35 w/w % of a binder,
- 5-25 w/w % of a filler, and optionally
- 0.1 - 5 w/w % of a fragrance,
wherein the w/w % fractions relate to the total weight of the lotion and the sum of the w/w % fractions is less or equal to 100 %.

In a further preferred embodiment of the invention, the cosmetic or personal care product is a loose powder. The loose powder preferably comprises approximately
- 10 - 30 w/w % of a pigment, in particular titanium dioxide,
- 20 - 50 w/w % of the inventive micropowder,
- 5 - 35 w/w % of a binder, in particular magnesium stearate,
- 5-25 w/w % of a filler, in particular talcum powder,
- 5 - 25 w/w % of a further pigment, and optionally
- 1-20 drops of a fragrance, in particular an essential oil.

In a further preferred embodiment of the invention, the cosmetic or personal care product is a liquid foundation. The liquid foundation preferably comprises
- 1 - 30 w/w % of an emulsifier,
- 1 - 20 w/w % of an emollient,
- 1 - 15 w/w % of a pigment,
- 0.05 - 5 w/w % thickener wax,
- 0.5 - 5 w/w % of a stabilizer,
- 1-10 w/w % of a humectant,
- 40 - 80 w/w % of a solvent,
- 0.5 - 25 w/w % of the inventive micropowder,
- 0.1 - 5 w/w % of an antioxidant, and
- 0.5 - 3 w/w % of a preservative,
wherein the w/w % fractions relate to the total weight of the lotion and the sum of the w/w % fractions is less or equal to 100 %.

In a further preferred embodiment of the invention, the cosmetic or personal care product is a liquid foundation. The liquid foundation preferably comprises approximately
- 15 w/w % of an emulsifier, in particular 4 w/w % polyglyceryl-3-polyricionate (PGPR) and 8 w/w % caprylic acid, 2 w/w % cetearyl alcohol and 1 w/w % Emulsan II with respect to the final product,
- 10 w/w % of an emollient, in particular sunflower oil,
- 4.65 w/w % of a pigment, in particular 0.35 w/w % of a red pigment, 0.10 w/w % of a black pigment, 0.70 w/w % of a yellow pigment and 3.50 w/w % titanium dioxide with respect to the final product,
- 0.75 w/w % of a first thickener wax, in particular carnauba wax,
- 0.25 w/w % of a second thickener wax, in particular xanthan gum,
- 1.5 w/w % of a stabilizer, in particular magnesium sulfate,
- 3 w/w % of a humectant, in particular glycerin,
- 61 - 63 w/w % of a solvent, in particular water,
- 1.5 w/w % of the inventive micropowder,
- 0.5 w/w % of an antioxidant, in particular tocopherol, and
- 1 w/w % of a preservative, in particular EUXYL PE 9010.

It may be preferred that the liquid foundation of the invention have a pH of approximately 6.35 and a viscosity of 33,000 cP, in particular 24 hours after production at 26 °C. Notably, the liquid foundation of the invention has a significantly higher viscosity than a liquid foundation using a synthetic petroleum-based polymer micropowder in place of the inventive micropowder.

In a further preferred embodiment of the invention, the cosmetic or personal care product is a butter cream. The butter cream preferably comprises
- 40 - 80 w/w % of a solvent,
- 1 - 20 w/w % of a humectant,
- 0.1 - 20 w/w % of the inventive micropowder,
- 0 - 5 w/w % of a rheology modifier,
- 5 - 20 w/w % of an emulsifier,
- 5 - 30 w/w % of an emollient,
- 0.01 - 5 w/w % of a fragrance,
- 0.5 - 3 w/w % of an antioxidant, in particular Vitamin E, and
- 0.01 - 3 w/w % of a preservative, in particular Biogard 221,
wherein the w/w % fractions relate to the total weight of the lotion and the sum of the w/w % fractions is less or equal to 100 %.

In a further preferred embodiment of the invention, the cosmetic or personal care product is a butter cream. The butter cream preferably comprises approximately
- 61 - 64 w/w % of a solvent, in particular water,
- 5 w/w % of a humectant, in particular glycerin,
- 0.2 - 1 w/w % of the inventive micropowder,
- 0 - 1 w/w % of a rheology modifier, in particular xanthan gum,
- 10 w/w % of an emulsifier, in particular comprising 4 w/w % Emulsan II and 6 w/w % cetearyl alcohol with respect to the final product,
- 20 w/w % of an emollient, in particular sunflower oil,
- 1 w/w % of a fragrance, in particular an essential oil,
- 0.5 w/w % of an antioxidant, in particular Vitamin E, and
- 0.2 w/w % of a preservative, in particular Biogard 221.

The essential oil used preferably comprises one or more of methyl glucose sesquistearate, lavender essential oil, orange essential oil, dehydroacetic acid and/or benzyl alcohol.

In a further preferred embodiment of the invention, the cosmetic or personal care product is a lipstick. The lipstick preferably comprises
- 5 - 50 w/w % of a structuring agent,
- 40 - 80 w/w % of an emollient,
- 1 - 20 w/w % of the inventive micropowder,
- 0.5 - 5 w/w % of a lake-dispersion agent,
- 0,1 - 3 w/w % of a pearling agent,
- 1-15 w/w % of one or more pigments,
- 0,01 - 3 w/w % of an antioxidant, and
- 0,01 - 3 w/w % of a preservative,
wherein the w/w % fractions relate to the total weight of the lotion and the sum of the w/w % fractions is less or equal to 100 %.

In a further preferred embodiment of the invention, the cosmetic or personal care product is a lipstick. The lipstick preferably comprises approximately
- 27.5 w/w % of a structuring agent, in particular comprising 11.5 w/w % synthetic wax, 2 w/w % euphorbia cerifera wax and 14 w/w % tricyclodecanemethyl isononanoate with respect to the final product,
- 62 - 63 w/w % of an emollient, in particular comprising 30,5 w/w % seed oil with respect to the final product,
- 4 w/w % of the inventive micropowder,
- 2 w/w % of a lake-dispersion agent, in particular hydrogenated polydecene and polyhydroxystearic acid,
- 0,7 w/w % of a pearling agent, in particular mica and iron oxide,
- 6 - 7 w/w % of one or more pigments,
- 0,1 w/w % of an antioxidant, in particular tocopherol and
- 0,75 w/w % of a preservative, in particular caprylyl glycol and phenoxyethanol.

Using the inventive micropowder, a matte or semi-matte lipstick with soft sensorial properties could be provided.

In a further preferred embodiment of the invention, the cosmetic or personal care product is a hand cream. The hand cream preferably comprises
- 40 - 80 w/w % of a solvent,
- 1-10 w/w % of a humectant,
- 0.1 - 3 w/w % of a thickener,
- 0.5 - 10 w/w % of the inventive micropowder,
- 3-10 w/w % of an emulsifier,
- 5 - 30 w/w % of an emollient,
- 0.1 - 10 w/w % of a fragrance,
- 0.1 - 3 w/w % of an antioxidant, and
- 0.1 - 3 w/w % of a preservative,
wherein the w/w % fractions relate to the total weight of the lotion and the sum of the w/w % fractions is less or equal to 100 %.

In a further preferred embodiment of the invention, the cosmetic or personal care product is a hand cream. The hand cream preferably comprises approximately
- 69 - 72 w/w % of a solvent, in particular water,
- 5 w/w % of a humectant, in particular glycerin,
- 0.2 w/w % of a thickener, in particular xanthan gum,
- 1 - 3 w/w % of the inventive micropowder,
- 6 w/w % of an emulsifier, in particular 1 w/w % Emulsan II and 5 w/w % cetearyl alcohol with respect to the final product,
- 15 w/w % of an emollient, in particular sunflower oil,
- 1 w/w % of a fragrance, in particular an essential oil,
- 0.5 w/w % of an antioxidant, in particular vitamin E, and
- 0.2 w/w % of a preservative, in particular EUXYL PE 9010.

In a further preferred embodiment of the invention, the cosmetic or personal care product is a lip cream. The lip cream preferably comprises
- 40 - 80 w/w % of a solvent,
- 1-10 w/w % of a humectant,
- 1-10 w/w % of a rheology modifier,
- 1 - 20 w/w % of the inventive micropowder,
- 5 - 30 w/w % of an emulsifier,
- 1 - 10 w/w % of a pigment,
- 0.1 - 10 w/w % of an active ingredient,
- 1 - 30 w/w % of an emollient, and
- 0.1 - 3 w/w % of a preservative,
wherein the w/w % fractions relate to the total weight of the lotion and the sum of the w/w % fractions is less or equal to 100 %.

In a further preferred embodiment of the invention, the cosmetic or personal care product is a lip cream. The lip cream preferably comprises approximately
- 66 - 67 w/w % of a solvent, in particular water,
- 6 w/w % of a humectant, in particular glycerin,
- 2.5 w/w % of a rheology modifier, in particular tapioca starch,
- 1.5 w/w % of the inventive micropowder,
- 13 w/w % of an emulsifier, in particular 11 w/w % cetearyl alcohol and 2 w/w % glyceril caprylate with respect to the final product,
- 2 w/w % of a pigment, in particular jojoba beads,
- 1.5 w/w % of an active ingredient, in particular D-panthenol,
- 6.5 w/w % of an emollient, in particular 0.5 w/w % veganolin, 4 w/w % vegetable oil and 2 w/w % dedraflow 30 with respect to the final product,
- 0.25 w/w % of a preservative and
- 0.2 w/w % tocopherol.

In a further preferred embodiment of the invention, the cosmetic or personal care product is a powder shampoo. The powder shampoo preferably comprises
- 10 - 40 w/w % of a surfactant,
- 40 - 65 w/w % of a filler,
- 1 - 30 w/w % of the inventive micropowder,
- 1-10 w/w % of a texture enhancer other than the micropowder,
- 1 - 7 w/w % of a conditioner agent,
- 0.1 - 3 w/w % of an emollient, and
- 0.1 - 3 w/w % of a fragrance,
wherein the w/w % fractions relate to the total weight of the lotion and the sum of the w/w % fractions is less or equal to 100 %.

In a further preferred embodiment of the invention, the cosmetic or personal care product is a powder shampoo. The powder shampoo preferably comprises approximately
- 32 w/w % of a surfactant, in particular 30 w/w % sodium cocoyl isethionate and 2 w/w% betaine with respect to the final product,
- 55 - 56 w/w % of a filler, in particular tapioca starch,
- 5 w/w % of the inventive micropowder as a texture enhancer,
- 5 w/w % of a further texture enhancer, in particular isoamyl laurate,
- 2 w/w % of a conditioner agent, in particular hydrotriticum,
- 0.5 w/w % of an emollient, in particular jojoba oil and
- 0.4 w/w % of a fragrance, in particular lavender oil.

The powder shampoo (also known as dry shampoo) of the invention was compared with a powder shampoo using a synthetic petroleum-based polymer micropowder in place of the inventive micropowder. After use of both powder shampoos, users reported a superior skin sensation after using the inventive powder shampoo.

In a further preferred embodiment of the invention, the cosmetic or personal care product is an anti-ageing cream. The anti-ageing cream preferably comprises approximately
- 40 - 80 w/w % of a first solvent, in particular water,
- 1-10 w/w % of a humectant,
- 0.1 - 5 w/w % of a rheology modifier,
- 0.01 - 2 w/w % of a complex agent,
- 2-10 w/w % of an emulsifier,
- 5 - 30 w/w % of an emollient,
- 0.5 - 5 w/w % of a skin-smoothing agent,
- 0.1 - 10 w/w % of a second solvent, in particular an organic solvent,
- 0.1 - 3 w/w % tocopherol,
- 0.5 - 10 w/w % of the inventive micropowder,
- 0.01 - 0.5 w/w % of a pH-adjuster, and
- optionally a fragrance,
wherein the w/w % fractions relate to the total weight of the lotion and the sum of the w/w % fractions is less or equal to 100 %.

In a further preferred embodiment of the invention, the cosmetic or personal care product is an anti-ageing cream. The anti-ageing cream preferably comprises approximately
- 63 - 64 w/w % of a first solvent, in particular water,
- 3 w/w % of a humectant, in particular propanediol,
- 1.8 w/w % of a rheology modifier, in particular xanthan gum,
- 0.1 w/w % of a complex agent, in particular sodium phytate aqua and alcohol,
- 6 w/w % of an emulsifier, in particular 4 w/w % palmitic acid and 2 w/w % cetearyl alcohol with respect to the final product,
- 20 w/w % of an emollient, in particular 3 w/w % shea butter, 2 w/w % squalane, 5 w/w % coco caprylate and 5 w/w % ethyhexyl stearate with respect to the final product,
- 1.5 w/w % of a skin-smoothing agent, in particular dimethicone,
- 1 w/w % of a second solvent, in particular hexylene glycol,
- 1.2 w/w % tocopherol,
- 1 w/w % of the inventive micropowder,
- 0.02 w/w % of a pH-adjuster, preferably comprising an aqueous sodium hydroxide solution of up to 30 w/w % concentration, and
- optionally a fragrance.

The inventive products and their formulations were subjected to extensive testing. It was found that the broad particle size distribution, the irregular shape of the bacterial cellulose particles and the high oil and water absorption contributed to improving the sensorial properties of the products and the user satisfaction. In particular, the products were found to have superior mattifying properties.

Due to the high mattifying effect, the micropowder could partially or completely replace talcum powder in formulations, making these safer and more satisfying to users. The products were also found to provide higher softness, lower tackiness and lower greasiness compared to like-for-like products comprising conventional micropowders instead of the inventive micropowder in their formulations. The micropowder could be used as a gentle peeling agent which agglomerates through rubbing. It was also found to be suitable for use in hot and cold products, ranging from make-up to cleaning products for use in hot water. The biocompatibility and sustainability of all products was improved by use of the inventive micropowder.

Terms such as substantially, approximately, about, approximately, nearly, etc. preferably describe a tolerance range of less than ± 20 %, preferably less than ± 10 %, particularly preferably less than ± 5 % and especially less than ± 1 % and include the exact value.

The average person skilled in the art will recognise that technical features, definitions and advantages of preferred embodiments of the micropowder according to the invention also apply to the method for producing it according to the invention, the products containing it, and vice versa.

### DETAILED DESCRIPTION OF THE INVENTION AND EXAMPLES

It should be understood that various alternatives to the embodiments of the invention described herein may be employed in practicing the invention. It is intended that the claims of the invention define the scope of the invention and that the method and apparatus within the scope of these claims and their equivalents be covered thereby.

Without intending to be limiting, the invention will be explained in more detail with reference to exemplary embodiments and the following figures:

### BRIEF DESCRIPTION OF THE FIGURES

Figs. 1 - Fig. 4 show magnified views of a first sample of micropowder according to a preferred embodiment of the invention, taken using SEM microscopy at steadily increasing levels of zoom.
Fig. 1 shows a magnified view of the first sample of the micropowder at 2 cm (scale) = 100 µm.
Fig. 2 shows a magnified view of the first sample of the micropowder at 2 cm (scale) = 30 µm (100x Zoom).
Fig. 3 shows a magnified view of the first sample of the micropowder at 2 cm (scale) = 10 µm (1000x Zoom).
Fig. 4 shows a magnified view of the first sample of the micropowder at 2 cm (scale) = 1 µm (10,000x zoom).
Figs. 5-6 show magnified views of a second sample of micropowder according to a further preferred embodiment of the invention, taken using SEM microscopy.

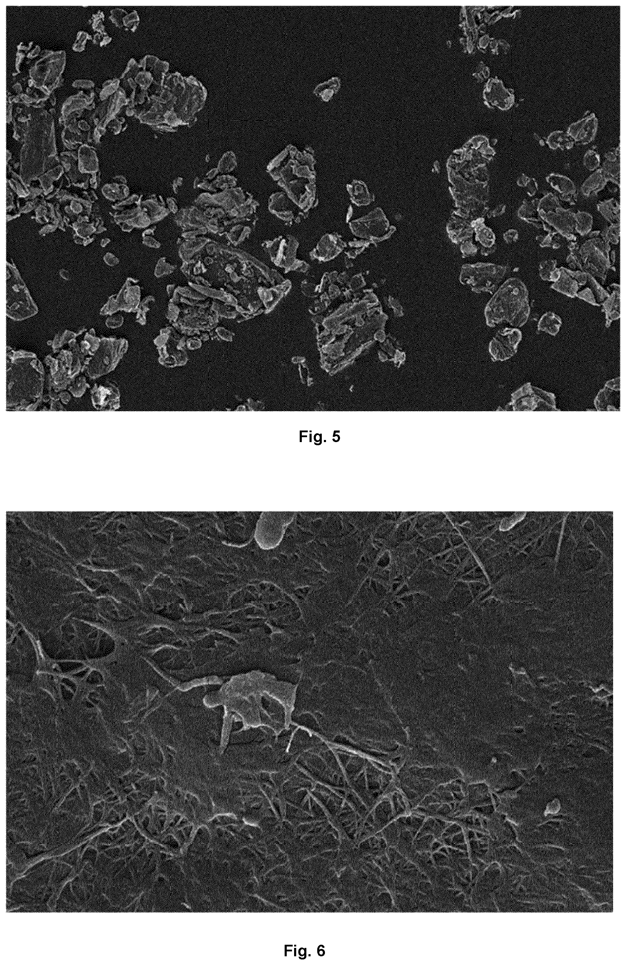

Fig. 5 shows a magnified view of the second sample of the micropowder at 1 cm (scale) = 30 µm (100x zoom).
Fig. 6 shows a magnified view of the second sample of the micropowder 1 cm (scale) = 1 µm (10,000x zoom).

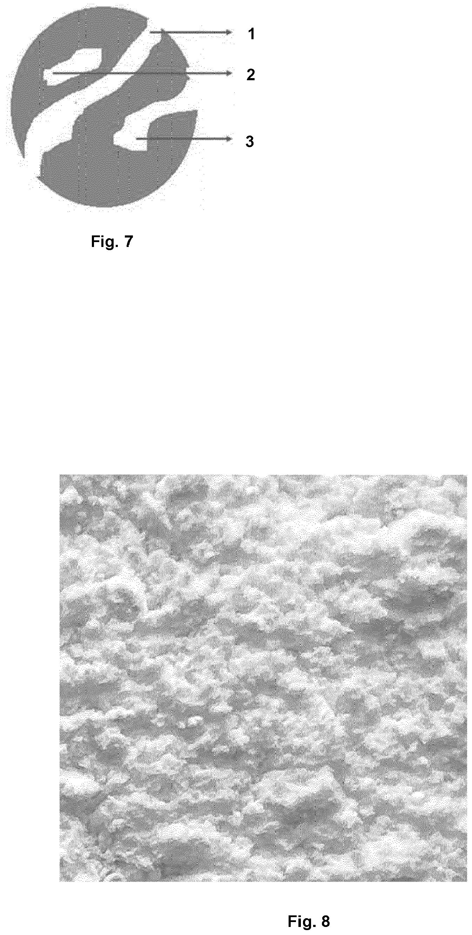

Fig. 7 is a schematic representation of the types of pores present in the particles of the micropowder sample.
Fig. 8 shows a photographic image of the micropowder of the preferred embodiment of the invention.

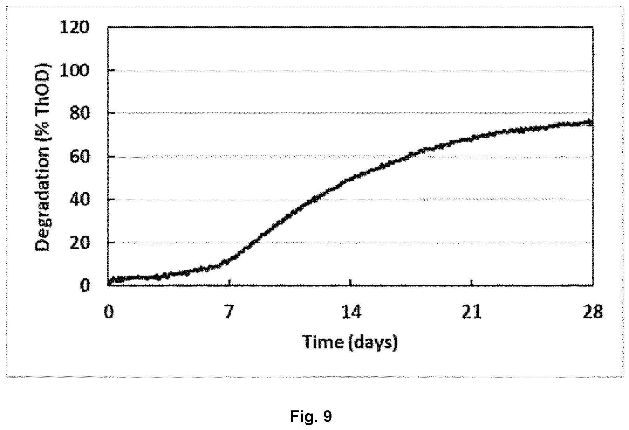

Fig. 9 shows a degradation of the micropowder of the preferred embodiment of the invention, indicating that 75 % biodegradation is surpassed by 28 days.

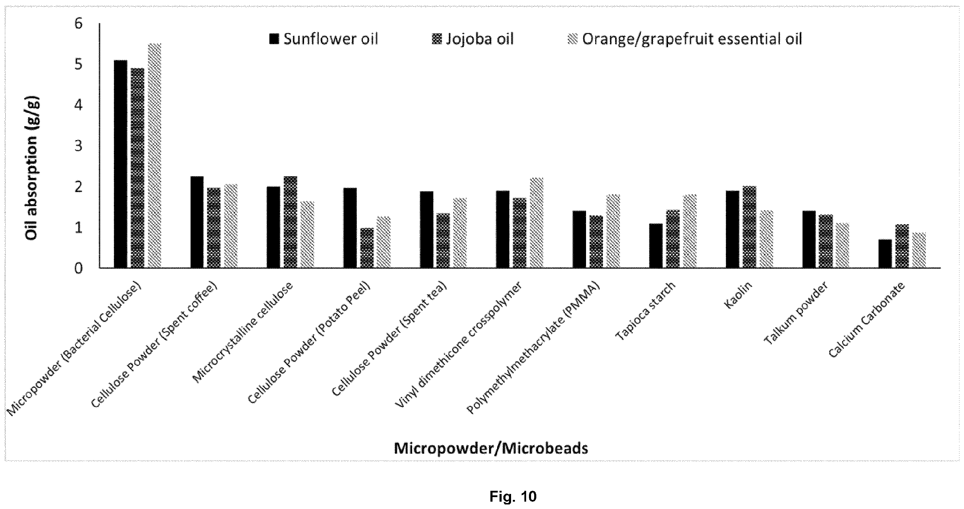

Fig. 10 shows oil absorption capacities of the micropowder of the preferred embodiment of the invention and a range of micropowders of the prior art for a range of different oils.

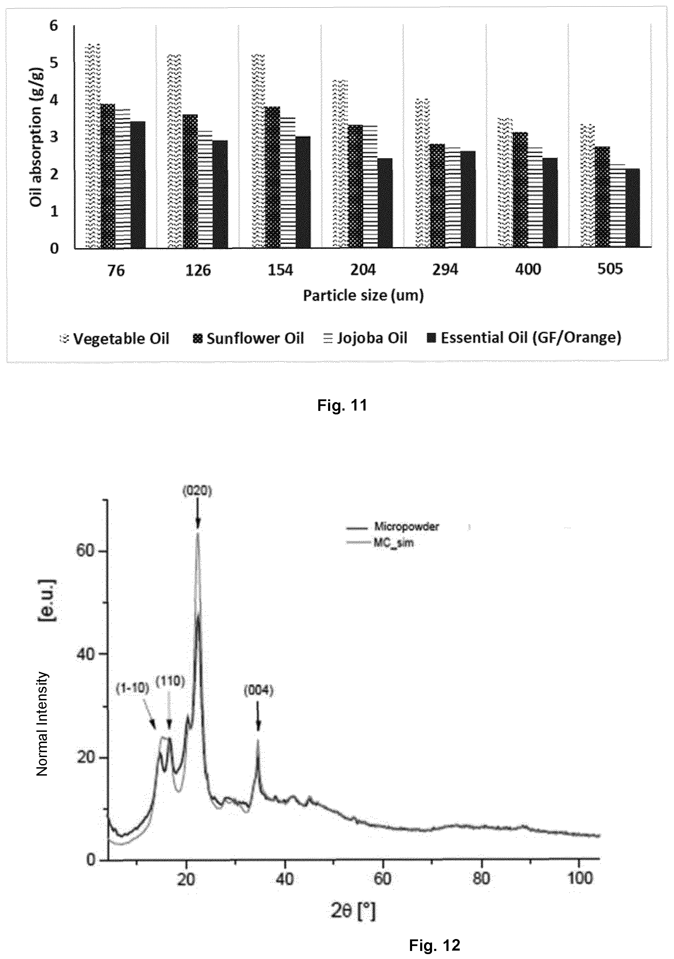

Fig. 11 shows oil absorption capacities for a number of micropowders according to various preferred embodiments of the invention, wherein the micropowders have varying average particle diameters.
Fig. 12 shows the results of an X-Ray diffraction analysis of the micropowder of the preferred embodiment.

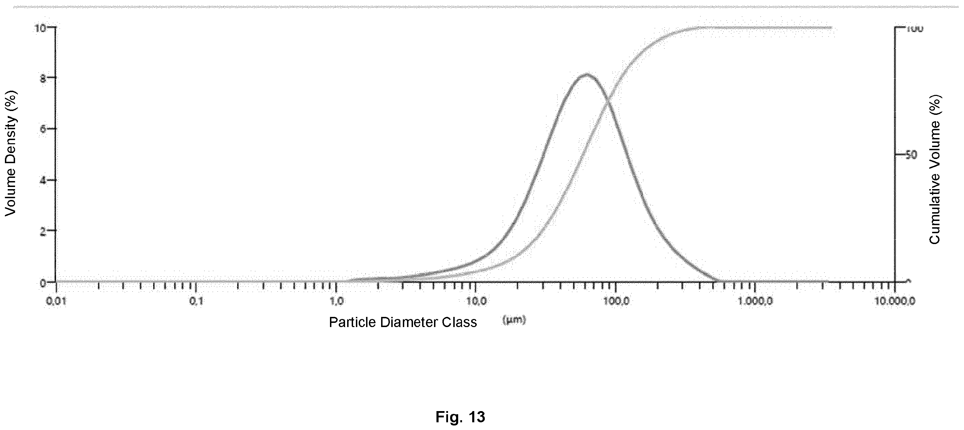

Fig. 13 shows the particle size distribution of the micropowder of the preferred embodiment.

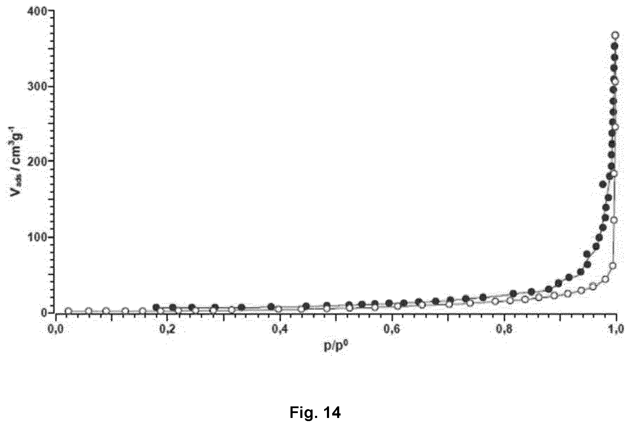

Fig. 14 shows the results of a volumetric gas adsorption analysis of the micropowder of the preferred embodiment.

### DETAILED DESCRIPTION OF THE PREFERRED EMBODIMENTS

The figures show the results of various tests carried out on a micropowder sample according to a preferred embodiment of the invention. The micropowder was formed from a number of bacterial cellulose pellicles harvested from a culture. After washing, the pellicles were dried at a relative humidity of 80 % at 30 °C for one week. The dried pellicles were treated with nitrogen, crushed and milling. The milling took place in steps, including cutting, course and fine milling. The finely milled bacterial cellulose was then mixed with deionized water and stirred to form a suspension having a concentration of approximately 2 w/w% bacterial cellulose. The suspension was then pumped to an atomizer and spray dried. High purity nitrogen gas was used to atomize the suspension. The spray drying took place in a B-290 Buchi spray dryer and the dried micropowder was then pumped to a cyclone before being collected.

The micropowder was then analysed by a scanning electron microscopy. A Gemini SEM 300 (Zeiss) device was used, having accelerating voltage 0,02 kV to 30 kV and a resolution of 0,8 nm at 15 kV. Magnifications of up to 2.000.000x were used to provide high-resolution images of the micropowder. The SEM-images were obtained using the InLens detector at 2 kV acceleration voltage and a probe current of 80 pA. A small amount of micropowder was applied onto aluminum stubs with carbon tape by using a spatula. To decrease the charging effect, a thin Cu-foil was applied at the edge of each Al-stub. Excess powder was blown off using air. A Polaron SC7640 sputter was used to coat the sample with gold. A coating sequence lasting for 40 s under 20 mA was used. Image analysis was carried out by image processing software (Olympus GmbH, Germany) to aid in the determination of particle sizes and their distribution.

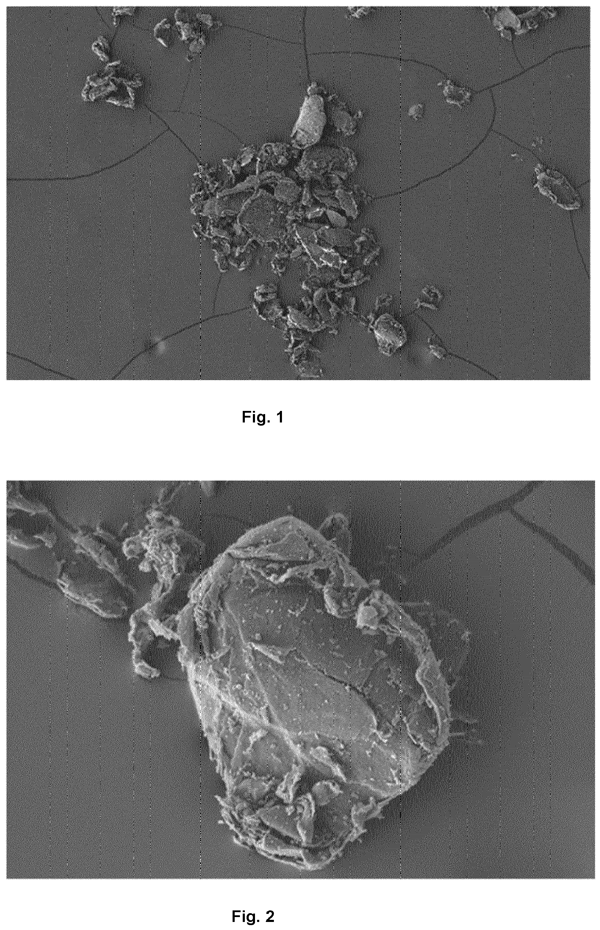

The results of a first sample at different magnifications are shown in Figs 1 - 4. As shown in Fig. 1, the bacterial cellulose particles had irregular forms including polyhedral and plate-like particles. The surfaces of the particles were uneven, which increased the surface area and the light diffusing properties.

In Fig. 2 various cracks and imperfections can be seen on the surface of a polyhedral particles. Adsorption testing was used to confirm that these were evidence of layers and of the presence of mesopores in the bacterial cellulose particles. As will be set out further below, adsorption analysis showed characteristics of Type III and Type V isotherms.

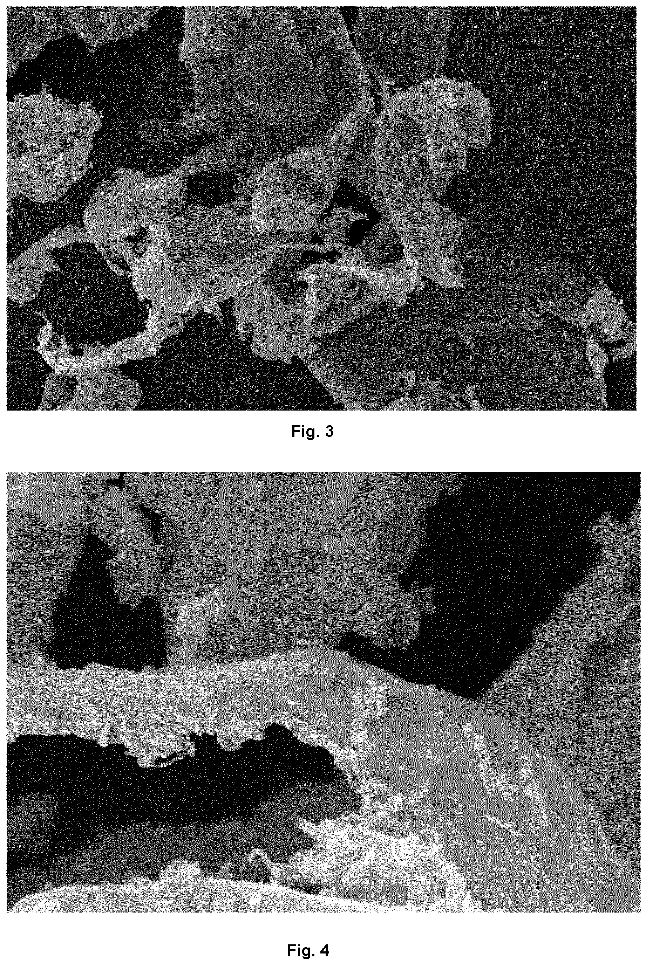

Fig. 3 shows a further enlarged image of the micropowder of the first sample, wherein the irregularity of the particle shapes, their layered structure and surface roughness is even more visible. It is clear from this figure that the bacterial cellulose particles of the micropowder have a larger surface area to mass ratio than conventional microbeads having relatively regular, spherical particles.

Fig. 4 shows a yet further enlarged image of the micropowder of the first sample, wherein the non-smooth, fibrous surface quality can be observed. This surface quality increases the surface area of the particles further which in turn improves their absorption and adsorption qualities.

Fig. 5 shows an enlarged image of a micropowder of a second sample. Plate-like, stratified particle can be observed. These can be packed tightly to provide high coverage and can scatter light to provide a matte finish.

The surface quality of these particles is shown in the further enlarged image of Fig. 6 (having the greatest enlargement of the SEM microscopy images reproduced herein). In this image, it can be observed that even apparently smooth portions of the bacterial cellulose particles display microvilli. These are visible as lines across the surface of the particles and are formed by cellulose fibers. Due to the presence of the microvilli at the surface of the bacterial cellulose particles, their surface area to volume ratio is dramatically increased, improving their absorption characteristics. Moreover, the overlapping and stratified arrangement of cellulose fibers leads to a large proportion of through and blind pores in the bacterial cellulose particles, which have openings at the particle surface. The absorption and adsorption capacities of the micropowder are hereby further increased, contributing to the astonishingly high performance of the inventive micropowder in comparison with the prior art.

Fig. 7 illustrates schematically the various types of pores found in the bacterial cellulose particles. Through pores (1) are channels passing between two openings in the surface of the bacterial cellulose particles. Blind pores (3) are channels having only a single opening in the surface of the bacterial cellulose whereas closed pores (2) are voids which are fully enclosed in the bacterial cellulose. Testing has shown that the bacterial cellulose particles of the micropowder had a high proportion of through pores which are thought to improve the oil absorption capacity of the micropowder.

Fig. 8 shows a photographic image of the micropowder. As can be seen, the micropowder has a neutral white color and is highly light scattering. It is noted that other samples had a range of colors, depending on their production methods, wherein the colors included white, off-white, pearl-white, cream, brown, yellow, pale yellow or dark yellow, dark brown or wood color without the use of pigments or bleaches. The neutral color and optical properties of the micropowder provided a high versatility for use as an opacifier or pigment in consumer products.

Fig. 9 shows the results of a biodegradability test carried out on the sample of the micropowder according to the preferred embodiment (shown in the SEM and photographic images described above). Using an OxiTop^{®} apparatus (WTW, Germany), microorganisms in a test bottle were left to convert oxygen to carbon dioxide (CO₂) during the biodegradation of the sample. OxiTop^{®} is a cap-shaped pressure meter attached to a test bottle and measures biochemical oxygen demand without generating any oxygen, simply by detecting the degree to which air pressure in the bottle decreases. After a test bottle was sealed with an OxiTop^{®} cap, the bottle was incubated in a thermostatic chamber (IN804, Yamato Scientific, Japan). According to the OECD (Guideline for Testing Chemicals: Ready biodegradability (1992)) and the CSCL (Guideline of Chemical Substances Control Law: Biodegradability Test of Chemical Substances by Microorganisms (2018) (in Japanese*))* guidelines, when the biochemical oxygen demand (BOD) value during a test period reaches 60% of the Theoretical Oxygen Demand (ThOD) for a sample, the sample is considered to be degraded completely (and the remaining 40% is assumed to have been assimilated by microorganisms). In the present sample, biodegradability of 75 % was achieved within 28 days.

Fig. 10 shows the results of oil absorption testing of a sample of the micropowder of the preferred embodiment and samples of various different micropowders of the prior art. The oil absorption capacity was tested in accordance with the ASTM D281-95 method, which is known to the skilled person. The results showed that the micropowder of the sample could absorb jojoba oil at a rate of approximately 5 g/g, whereas the absorption rate of sunflower oil and orange and grapefruit essential oil both exceeded 5 g/g. These results more than doubled the performance of the most commonly used prior art micropowders. As can be seen in the chart, the closest performance was provided by microcrystalline cellulose particles from plant cellulose. Due to its different material properties, its oil absorption capacity was approximately 2 g/g for sunflower oil under the same conditions. This was less than 40 % of the absorption capacity of the micropowder produced according to the invention.

The oil absorption performance of the micropowder of the invention even further outstripped that of the metallic and plastic micropowders. Combined with the biodegradability performance demonstrated above, it is clear that the inventive micropowder can replace the environmentally problematic micropowders of the prior art without sacrificing consumer satisfaction.

Further analysis results for the sample used are set out Table 1 below.

**Table 1: Analysis results of the micropowder according to a preferred embodiment of the invention**

| **Properties** | | **Micropowder of the Invention** |
|---|---|---|
| Source | | Bacterial Cellulose |
| Biodegradability (28 d) ^{a} | | 75% |
| Water holding capacity | | >95% |
| Dosage (%) | | 1.0-5.0 |
| Microscopy (SEM) | | Irregular |
| Crystallinity (%) ^{b} | | 30-95 |
| pH (10% aq) | | 9.0-9.5 |
| Particle size (µm) ^{c} | D₅₀ | 64.7 |
| | D₉₀ | 182.7 |
| Moisture content (%) ^{d} | | 4.0-5.0 |
| Bulk density (g/cm³) | | 161 |
| Oil absorption (g/g of sample) ^{e} | | 2.5-9.7 |
| Water absorption (g/g of sample) ^{f} | | 3-12 |
| Surface Area (m²/g) ^{g} | | 2-25 |
| Degree of Polymerization ^{h} | | 1000-20000 |

| | | |
|---|---|---|
| ^{a} Monochromatic respiration test (OxiTop^{®} Control measuring system from WTVη, ^{b} dual-circuit diffractometer (D5000, Bruker-AXS), ^{c} ULM Standard PIDS, ^{d}KERN DAB 200-3 (moisture meter), ^{e} Modified ASTM D2819-5 method, ^{f} Dynamic vapor sorption, 20 °C, 76 % humidity, ^{g} BET nitrogen adsorption isotherms, ^{h} GPC, 8% LiCI in DMAc, FM A (0.5% LiCl in DMAc). | | |

Fig. 11 shows a comparison of the oil absorption capacities of a number of micropowders produced according to the invention, wherein the micropowders have differing average diameters D₅₀. The oil absorption capacities of the samples were tested in accordance with ASTM D281-95 under like-for-like conditions. It can be seen that for all of the samples tested, despite their different particle sizes, the oil absorption capacity was high, being able to outstrip that of the prior art micropowders shown in the previous figure.

Moreover, Fig. 11 shows that lower particle sizes favour a higher oil absorption for all oils. In particular for vegetable oils, a strong correlation between absorption capacity and average particle diameter can be observed. The particle size can thus be used to adjust this property of the micropowder, e.g. by adjusting the grinding speed and/or duration.

Fig. 12 shows the results of an x-ray diffraction analysis of the micropowder sample of the preferred embodiment (the properties of which are also listed in Table 1 above). The x-ray diffraction analysis was carried out using a dual-circuit diffractometer (D5000, Bruker-AXS), Monochromator: Ge (111) primary beam monochromator, Wavelength: Cu-Kα1; λ = 0.15406 nm, Detector: Scintillation counter, Operation (standard): symmetrical transmission. Diffraction angles of 5-80 deg (2θ) were analysed in steps of 0.02 deg with 0.2 s per step while rotating the sample. Prior to the analysis, samples were ground with ethanol, dispersed onto zerobackground silicon sample holders and placed under a lamp to evaporate the ethanol. The results were used to study the crystallinity of the micropowder.

Fig. 13 shows the results of a particle size distribution analysis of the micropowder sample of the preferred embodiment. The particle size distribution was measured using laser diffraction with the Mastersizer 3000 (Malvern Panalytical, Ltd, Malvern, Great Britain), using a dry method with an Aero S accessory. The light scattering data, converted to particle size distribution were analyzed using an Mie-scattering model, using the non-spherical particle type and MgCCb as material (i.e. MgCCb settings for the refractive index (1.717) and adsorption index (0.01)). Prior to adding the sample to the instrument, the sample container was mixed well in order to ensure good sampling. A few grams of powder were used for each measurement, the measurement time was set to 10 - 30 seconds. The lower obstruction limit was set to 0.5% and the upper limit to 5%, the air pressure was set to 1.5 bar. During the measurement the feed rate was constantly adjusted so that the obstruction was kept between 0.5 and 5%. All measurements were averaged and run at least five times. The sample was found to have an average particle diameter D₅₀ of 64.7 µm as set out in Table 1. This provided excellent oil absorption, water absorption, matte finish and coverage of pores whilst being safe and biocompatible.

Fig. 14 shows the results of a volumetric gas adsorption analysis carried out on the sample of the micropowder. This was used to carry out a Brunauer-Emmett-Teller (BET) analysis of the surface area to mass ratio of the micropowder and to draw conclusions about its porosity. The test including nitrogen gas adsorption in a liquid nitrogen bath at 77 K. Measurements were made on a The Belsorp Max II surface area and porosity analyser. Prior to analysis, samples were degassed for 12 hours at 105°C under nitrogen gas flow in a FlowPrep060 Sample Degas System. The surface area was determined by using the well-recognized BET equation, and hence calculated from the nitrogen sorption isotherms (Brunauer et al, JACS, 60, 1938, 309-319).

The data shows that the analyzed micropowder corresponds to a type V isotherm and is mesoporous. Type V isotherms are commonly observed for flat, homogeneous adsorbents. The initial pathway of this isotherm is similar to that of Type III. In this instance, the adsorbate preferentially interacts with the monolayer than the adsorbent surface, due to the lower heat of adsorption compared to the heat of liquefaction. A high affinity isotherm meanwhile is typical for very strong adsorption interactions.

From the adsorption data, an average surface area to mass ratio S_{BET} of 7.8 m²/g was calculated for the sample. An average surface area to mass ratio of mesoporous particles S_{Meso} of 42.1 m²/g and an average pore radius rₚₒᵣₑ of 5.6 nm were calculated.

Table 2 shows the results of user testing of various formulations applied to the skin.

**Table 2 - Results of Sensory Assessment of the Micropowder**

| **Property** | **Inventive Micropowder (Bacterial Cellulose)** | **Synthetic Polymer (PMMA)** | **Mineral (Silica)** |
|---|---|---|---|
| Softness | 6.3 | 5.2 | 5.3 |
| Greasiness | 2.8 | 5.2 | 3.7 |
| Stick (After-Feel) | 1.8 | 6.2 | 4.2 |
| Soft (After-Feel) | 5.5 | 3.2 | 3.7 |
| Greasy (After-Feel) | 3.5 | 5.3 | 3.5 |

The micropowder was tested in the form of a lotion having a formulation as set out in Table 3 below. 70 µL of the lotion were applied to the back of users' hands and spread with three fingers of the other hand 8 times. Users were asked to evaluate the feeling during application for softness and greasiness. The scale used was from 0 to 10 wherein 0 represents non-softness and non-greasiness and 10 represents very high softness and very high greasiness respectively.

The after-feel of the micropowder was tested using a lotion having a formulation as set out above. 70 µL of the lotion were applied to the back of users' hands and spread with three fingers of the other hand 20 times. Users were asked to score the after-feel after one minute. The scale used for assessing the stickiness of the after-feel was from 0 to 10, wherein 0 represents non-stickiness and 10 represents a very high degree of stickiness. The stickiness was tested by applying light taps to the back of the hand wearing the lotion using the other hand.

The softness of the after-feel was tested by users stroking the back of their hands. The scale used was from 0, which represents non-softness to 10 which represents a very high softness.

The greasiness of the after-feel was tested by users passing their index fingers and thumb over the back of the hands wearing the lotion. The scale used was from 0 to 10 wherein 0 represented non-greasiness and 10 represented a very high degree of greasiness.

Users reported a softer feeling both during and after wear of the formulation comprising the inventive bacterial cellulose micropowder in comparison with formulations comprising polymethylmethacrylate or silica micropowders. Users also reported less stickiness and less greasiness. Visually, a semi-matte effect could be observed when the micropowder of the preferred embodiment was used. It is thought that this is due to the high surface area to volume ratio, high porosity and irregular shape of the bacterial cellulose particles which provided a high oil absorption. The particle size distribution was also found to provide good coverage when used in foundations as well as providing volume and length to eyelashes when used in a mascara. The micropowder therefore shows a great potential to replace the micropowders of the state of the art.

The micropower has proven in particular advantageous for use in a cosmetic or personal care product. Without intending to be limiting, the following cosmetic formulations set out in Tables 3-12 exemplify possible applications and the extraordinary potential of the inventive micropowder in improving the properties of the respective products.

**Table 3 - Exemplary formulation of a lotion for the body or face, comprising the inventive micropowder as a sensory enhancer and comparison with equivalent formulations using other powders:**

| **Ingredients** | **Material Function** | **Micropowder (I) (Bacterial Cellulose) (% w/w)** | **Synthetic Polymer Powder (II) (Petroleum Based) (%** w/w) | **Mineral Powder (III) (Silica) (% w/w)** |
|---|---|---|---|---|
| Water | Solvent | 76.05 | 76.05 | 76.05 |
| Glycerine | Humectant | 5 | 5 | 5 |
| Xanthan gum | Thickener | 0.2 | 0.2 | 0.2 |
| *Powder (IlIIlIII)* | Sensory Enhancer | 1.5 | 1.5 | 1.5 |
| Oils | Emollient | 10 | 10 | 10 |
| Emulsifiers | Emulsifier | 5.5 | 5.5 | 5.5 |
| Tocopherol | Antioxidant | 0.5 | 0.5 | 0.5 |
| Euxyl pe9010 | Preservative | 1 | 1 | 1 |
| Essential oil | Fragrance | 0.25 | 0.25 | 0.25 |

| **Performance** | | | | |
|---|---|---|---|---|
| pH | | 6.90 | 6.67 | 6.56 |
| Viscosity (cP) (after 24 h, 26 ±1 °C) | | 24,584 | 17,866 | 18,567 |

**Table 4 - Exemplary formulation of a mascara comprising the inventive micropowder:**

| **Ingredients** | **Material Function** | **Fraction (% w/w)** |
|---|---|---|
| Propylene glycol | Humectant | 3 |
| Xanthan gum | Rheology modifier | 0.3 |
| Water | Solvent | 60 |
| Black pigment | Pigment | 9 |
| Beeswax | Structuring agent | 8 |
| Soya wax | Structuring agent | 0 |
| Carnauba wax | Structuring agent | 4 |
| Cetearyl alcohol | Emulsifier | 3 |
| Stearic acid | Emulsifier | 5 |
| *Micropowder* (Bacterial Cellulose) | Cellulose | 2 |
| EUXYL PE 9010 | Preservative | 1 |
| Tocopherol | antioxidant | 0.05 |
| Sodium Hydroxide | pH adjuster | pH adjustment |
| pH | | 7 |
| Viscosity (cP) (after 24 h, 25 ±2 °C) | | 433,920 |
| Appearance | | Black waxy emulsion |

**Table 5 - Exemplary formulations and comparison of four loose powders comprising the inventive micropowder:**

| **Ingredients** | **Material Function** | **Benchmark (% w/w)** | **Formualtion Ex-5 (% w/w)** | **Formualtion Ex-6 (% w/w)** | **Formualtion Ex-7 (% w/w)** | **Formualtion Ex-8 (% w/w)** |
|---|---|---|---|---|---|---|
| Titanium dioxide | Pigment | 12 | 12 | - | 20 | - |
| *Micropowder* (Bacterial Cellulose) | Texturing agent | - | 28 | 40 | 43 | 50 |
| Magnesium stearate | Stabilizer/ Binder | 20 | 20 | 20 | 7 | 30 |
| Talcum powder | Filler | 58 | 20 | 20 | 20 | - |
| Colour | Pigment | 10 | 20 | 20 | 10 | 20 |
| Essential oil (4 drops) | Fragrance | yes | - | - | - | - |
| Essential oil (8 drops) | Fragrance | - | - | yes | - | - |
| Essential oil (16 drops) | Fragrance | - | yes | - | - | yes |
| Sensory test | | Very Good | Excellent | Good | Excellent | Good |

**Table 6 - Exemplary formulation of a liquid foundation comprising the inventive micropowder and comparison with liquid foundations comprising different powders:**

| **Ingredients** | **Material Function** | **Formulation comprising Micropowder (I) (Bacterial Cellulose) (% w/w)** | **Formulation comprising Synthetic Polymer Powder (II) (Petroleum Based) (% w/w)** | **Formulation comprising Mineral Silica Powder (III) (% w/w)** |
|---|---|---|---|---|
| Polyglyceryl-3 polyricionate (PGPR) | Emulsifier | 4.00 | 4.00 | 4.00 |
| Caprylic acid | Emulsifier | 8.00 | 8.00 | 8.00 |
| Sunflower oil | Emollient | 10.00 | 10.00 | 10.00 |
| Red pigment | Pigment | 0.35 | 0.35 | 0.35 |
| Black pigment | Pigment | 0.10 | 0.10 | 0.10 |
| Yellow pigment | Pigment | 0.70 | 0.70 | 0.70 |
| Titanium dioxide | Pigment | 3.50 | 3.50 | 3.50 |
| Cetearyl alcohol | Emulsifier | 2.00 | 2.00 | 2.00 |
| Emulsan II | Emulsifier | 1.00 | 1.00 | 1.00 |
| Carnaubawax | Thickener wax | 0.75 | 0.75 | 0.75 |
| Magnesium sulfate | Stabiliser | 1.50 | 1.50 | 1.50 |
| Glycerin | Humectant | 3.00 | 3.00 | 3.00 |
| Xantham gum | Thickener wax | 0.25 | 0.25 | 0.25 |
| Water | Solvent | 61.85 | 63.35 | 63.35 |
| *Powder (IlIIlIII)* | Skin feel enhancer | 1.50 | 1.50 | 1.50 |
| Tocopherol | Antioxidant | 0.50 | 0.50 | 0.50 |
| Euxyl pe9010 | Preservative | 1.00 | 1.00 | 1.00 |

| **Performance** | | | | |
|---|---|---|---|---|
| pH | | 6.35 | 6.40 | 6.08 |
| Viscosity (cP) after 24 h, 26 ±1 °C | | 32,944 | 29,389 | 36,016 |
| Appearance | | Tinted cream | Cream | Cream |

**Table 7 - Exemplary formulations and comparison of various butter cream formulations Ex-12 to Ex-18 with and without the inventive micropowder:**

| **Ingredients** | **Material Function** | **Ex-12 (% w/w)** | **Ex-13 (% w/w)** | **Ex-14 (% w/w)** | **Ex-15 (% w/w)** | **Ex-16 (% w/w)** | **Ex-17 (% w/w)** | **Ex-18 (% w/w)** |
|---|---|---|---|---|---|---|---|---|
| Water | Solvent | 62.8 | 62.3 | 62.8 | 62.6 | 63.1 | 62.3 | 61.8 |
| Glycerin | Humectant | 5 | 5 | 5 | 5 | 5 | 5 | 5 |
| *Micropowder* (Bac. Cellulose) | Sensory enhancer | - | 0.5 | 0.5 | 0.2 | 0.2 | 1 | 1 |
| Xantham Gum | Rheology Modifier | 0.5 | 0.5 | 0 | 0.5 | 0 | 0 | 0.5 |
| Emulsan II ¹ | Emulsifier | 4 | 4 | 4 | 4 | 4 | 4 | 4 |
| Cetearyl Alcohol | Emulsifier | 6 | 6 | 6 | 6 | 6 | 6 | 6 |
| Sunflower Oil | Emollient | 20 | 20 | 20 | 20 | 20 | 20 | 20 |
| Essential oil ² | Fragrance | 1 | 1 | 1 | 1 | 1 | 1 | 1 |
| Vitamin E | Antioxidan t | 0.5 | 0.5 | 0.5 | 0.5 | 0.5 | 0.5 | 0.5 |
| Biogard 221 ³ | Preservati ve | 0.2 | 0.2 | 0.2 | 0.2 | 0.2 | 0.2 | 0.2 |
| pH | | 7.47 | 7.70 | 7.92 | 7.79 | 7.98 | 7.52 | 7.54 |
| Viscosity (cP) after 24 h, 26 ±1 °C | | 92,997 | 78,225 | 58,721 | 97,898 | 79,794 | 79,314 | 76,464 |

| | | | | | | | | |
|---|---|---|---|---|---|---|---|---|
| *1: Methyl glucose Sesquistearate, 2: Lavener and Orange, 3*: *Dehydroacetic Acid, Benzyl Alcohol* | | | | | | | | |

**Table 8 - Exemplary formulation of a lipstick comprising the inventive micropowder:**

| **Ingredients** | **Material Function** | **Formulation (% w/w)** |
|---|---|---|
| Synthetic wax | Structuring agent | 11.5 |
| Euphorbia cerifera Wax | Structuring agent | 2 |
| Tricyclodecanemethyl isononanoate | Structuring agent | 14 |
| Emollient | Emollient | 32.1 |
| Seed oil | Emollient | 30,5 |
| *Micropowder* (Bacterial Cellulose) | Texturizing agent/skin feel enhancer | 4 |
| Hydrogenated polydecene, Polyhydroxystearic Acid | Lake -dispersion agent | 2 |
| Mica, iron oxide | Pearl | 0,7 |
| Pigments | Pigments | 6.36 |
| Tocopherol | Antioxidant | 0,1 |
| Caprylyl glycol, phenoxyethanol | Preservative | 0,75 |

**Table 9 - Exemplary formulations and comparison of hand creams with and without the inventive micropowder:**

| **Ingredients** | **Material Function** | **Formulation comprising Micropowder (I) (Bacterial Cellulose) (% w/w)** | | | **Formulation comprising Tapioca starch (% w/w)** | **Formulation comprising Vinyl Dimethicone (% w/w)** |
|---|---|---|---|---|---|---|
| | | **Ex-20** | **Ex-21** | **Ex-22** | **Ex-23** | **Ex-24** |
| Water | Solvent | 71.1 | 70.1 | 69.1 | 70.1 | 70.1 |
| Glycerin | Humectant | 5 | 5 | 5 | 5 | 5 |
| Xanthan gum | Thickener | 0.2 | 0.2 | 0.2 | 0.2 | 0.2 |
| *Powder (I) or Comparison* | Sensory Enhancer | 1 | 2 | 3 | 2 | 2 |
| Xantham Gum | Thickener | 0.2 | 0.2 | 0.2 | 0.2 | 0.2 |
| Emulsan II | Emulsifier | 1 | 1 | 1 | 1 | 1 |
| Cetearyl Alcohol | Emulsifier | 5 | 5 | 5 | 5 | 5 |
| Sunflower Oil | Emollient | 15 | 15 | 15 | 15 | 15 |
| Essential oil | Fragrance | 1 | 1 | 1 | 1 | 1 |
| Vitamin E | Antioxidant | 0.5 | 0.5 | 0.5 | 0.5 | 0.5 |
| EUXYL PE 9010 | Preservative | 0.2 | 0.2 | 0.2 | 0.2 | 0.2 |

| **Performance** | | | | | | |
|---|---|---|---|---|---|---|
| Stability (centrifugation test) 24 h | | Stable | Stable | Stable | Stable | Stable |
| pH after 24 h, 26 ±1 °C | | 7.04 | 7.44 | 6.96 | 6.72 | 6.60 |
| Viscosity (cP) after 24 h, at 26 ±1 °C | | 29,867 | 20,610 | 3,474 | 31,430 | 3,577 |

**Table 10 - Exemplary formulation of a lip cream comprising the inventive micropowder:**

| **Ingredients** | **Material Function** | **Formulation Ex-25 (% w/w)** |
|---|---|---|
| Water | Solvent | 66.55 |
| Glycerin | Humectant | 6 |
| Tapioca Starch | Rheology Modifier | 2.5 |
| *Micropowder* (Bacterial Cellulose) | Texturing agent | 1.5 |
| Cetearyl Alcohol | Emulsifier | 11 |
| Glyceril Caprilate | Emulsifier | 2 |
| Jojoba Beads | Pigments | 2 |
| D-Panthenol | Active ingredient | 1.5 |
| Veganolin | Emollient | 0.5 |
| Vegetable Oil | Emollient | 4 |
| Dedraflow 30 | Emollient | 2 |
| Preservative | Preservative | 0.25 |
| Tocopherol | Tocopherol | 0.2 |

**Table 11 - Exemplary formulation of a powder shampoo comprising the inventive micropowder and comparison with a powder shampoo comprising a synthetic powder.**

| **Ingredients** | **Material Function** | **Formulation comprising Micropowder (I) (Bacterial Cellulose) (% w/w)** | **Formulation comprising Synthetic Polymer Powder (II) (Petroleum Based) (% w/w)** |
|---|---|---|---|
| | | **Ex-26** | **Ex-27** |
| Sodium Cocoyl Isethionate | Surfactant - Cleaning agent | 30 | 30 |
| Tapioca Starch | Filler | 55.1 | 55.1 |
| *Powder (IlII)* | Texture enhancer | 5 | 5 |
| Isoamyl Laurate | Texture enhancer | 5 | 5 |
| Hydrotriticum | Conditioner agent | 2 | 2 |
| Betaine | Surfactant - Cleaning agent | 2 | 2 |
| Jojoba Oil | Emollient | 0.5 | 0.5 |
| Lavender oil | Fragrance | 0.4 | 0.4 |

| **Performance** | | | |
|---|---|---|---|
| Appearance | | White Powder | White Powder |
| Sensory Quality (After Washing) | | Superior | Good |

**Table 12 - Exemplary formulation Ex-28 of an anti-ageing cream comprising the inventive micropowder**

| **Ingredients** | **Material Function** | **Formulation Ex-28 (% w/w)** |
|---|---|---|
| Aqua/water | Solvent | 63.38 |
| Propanediol | Humectant | 3 |
| Xanthan gum | Rheology modifier | 1.8 |
| Sodium Phytate Aqua; Alcohol | Complex agent | 0.1 |
| Palmitic acid | Emulsifier | 4 |
| Cetearyl alcohol | Emulsifier | 2 |
| Shea butter | Emollient | 3 |
| Squalane | Emollient | 2 |
| Coco caprylate/caprate | Emollient | 5 |
| Ethyhexyl stearate | Emollient | 5 |
| Dimethicone | Skin-smoothing | 1.5 |
| Hexylene glycol | Solvent | 1 |
| Tocopherol | Antioxidant | 1.2 |
| *Micropowder* (Bacterial Cellulose) | Texture Enhancer | 1 |
| Parfum/fragrance | Fragrance | qs |
| Sodium hydroxide, water (30%) | pH Adjuster | 0.02 |

| **Performance** | | |
|---|---|---|
| pH | | 5.3 |
| Viscosity (cP) (after 24 h, 25 ±2 °C) | | 6864 |
| Appearance | | Pink emulsion |

## Claims

1. Micropowder comprising bacterial cellulose particles,
**characterized in that**
the micropowder has an oil absorption capacity of at least 2.5 g/g and is biodegradable.

2. Micropowder according to claim 1
**characterized in that**
the micropowder is biodegraded to 75% within 35 days, preferably within 28 days.

3. Micropowder according to any of the previous claims
**characterized in that**
the micropowder has an oil absorption capacity of at least 4 g/g, preferably at least 4.5 g/g, even more preferably at least 5 g/g.

4. Micropowder according to any of the previous claims
**characterized in that**
an average particle diameter D₅₀ of the bacterial cellulose particles is between 1 - 1000 µm and an average crystallinity of the bacterial cellulose particles is between 30 - 80%.

5. Micropowder according to any of the previous claims
**characterized in that**
the micropowder is produced by a method which includes grinding bacterial cellulose from a culture and drying the ground bacterial cellulose, wherein the drying is preferably configured to cause hornification of the bacterial cellulose.

6. Micropowder according to any of the previous claims
**characterized in that**
the average particle diameter D₅₀ of the bacterial cellulose particles is between 1 - 500 µm, preferably between 5 - 250 µm and especially preferably between 25 - 100 µm, wherein the bacterial cellulose particles preferably have an average aspect ratio between 10:1 and 1:10, preferably between 5:1 and 1:5, more preferably between 2:1 and 1:2.

7. Micropowder according to any of the previous claims
**characterized in that**
an average crystallinity of the bacterial cellulose particles is between 40 - 80%, preferably between 40 - 60%, especially preferably between 40 - 50%.

8. Micropowder according to any of the previous claims
**characterized in that**
the bacterial cellulose particles are mesoporous with an average pore radius of 1 - 50 nm, preferably 5 - 20 nm, even more preferably 7 nm, wherein the surface area to mass ratio of the bacterial cellulose particles is preferably 10 - 100 m²/g, more preferably 10 - 80 m²/g.

9. Method for producing a micropowder according to any of the previous claims,
**characterized in that** the method comprises the following steps:
- producing bacterial cellulose from a bacterial culture such that an average crystallinity of the bacterial cellulose produced is between 30 - 80%,
- grinding bacterial cellulose to form bacterial cellulose particles with an average particle diameter of 1 - 1000 µm,
- drying the ground bacterial cellulose particles.

10. Method according to the previous claim
**characterized in that**
the bacterial cellulose is at least partially dried between its production and grinding, wherein partial drying preferably occurs in an atmosphere having a relative humidity of up to 85 %, preferably up to 80 %, and a temperature between 20 - 40 °C, preferably 25 - 35 °C, for a period of 3 - 10 days, preferably for a period of 5 - 9 days.

11. Method according to any of claims 9 or 10
**characterized in that**
the bacterial cellulose is treated with a liquified gas, preferably liquid nitrogen, before grinding.

12. Method according to any of claims 9-11
**characterized in that**
before drying, the ground bacterial cellulose particles are mixed with deionised water to form a suspension having a concentration of 0.5 - 5 w/w% of bacterial cellulose in water, more preferably having a concentration of 1.0 - 3.5 w/w%.

13. Method according to the previous claim
**characterized in that**
the suspension of bacterial cellulose in water is spray dried, wherein the spray drying preferably comprises the steps of:
- atomising the suspension of bacterial cellulose in water to form droplets suspended in a gas, wherein the atomisation is preferably aided by use of a pressurised gas, in particular air or an inert gas, especially preferably nitrogen,
- subjecting the suspended droplets to a flow of heated air, such that the water content of the droplets is reduced, the heated air preferably having a temperature between 80 - 250 °C, especially preferably 150 - 190 °C,
- collecting the dried particles.

14. Method according to any of claims 9-13
**characterized in that**
the suspension of bacterial cellulose in water is freeze-dried, wherein the freeze-drying preferably comprises the steps of:
- freezing the suspension at a temperature between -10 and -50 °C, preferably between -15 and -25 °C,
- reducing the pressure of a controlled atmosphere surrounding the frozen suspension to less than 50 mbar, preferably less than 1 mbar, more preferably less than 0.5 mbar, such that ice from the frozen suspension is sublimated and a resulting cake has a moisture content of less than 7 w/w%, preferably less than 5 w/w% and especially preferably less than 1 w/w%.

15. Cosmetic or personal care product comprising the micropowder of any of claims 1-8, wherein the cosmetic or personal care product is preferably an anti-ageing product, a treatment product for oily and acne-prone skin , a face mask, a moisturizer, a buttercream, a lotion, a foundation, a concealer, a loose or pressed powder, a mascara, a lipstick, a lip gloss, a fluid shampoo, a dry shampoo, a shampoo bar, a hair mask, a hair conditioner, a hair styling product, an anti-perspirant, a deodorant or an oral care product.
